(19) Europäisches Patentamt
European Patent Office
Office européen des brevets

(11) EP 2 992 880 A1

# (12) EUROPEAN PATENT APPLICATION

(43) Date of publication:
**09.03.2016 Bulletin 2016/10**

(21) Application number: **14183542.1**

(22) Date of filing: **04.09.2014**

(51) Int Cl.:
***A61K 31/404*** *(2006.01)* ***A61K 31/555*** *(2006.01)*
***A61K 33/00*** *(2006.01)* ***A61K 45/06*** *(2006.01)*
***A61P 25/04*** *(2006.01)* ***A61P 29/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Fundació Institut de Recerca de l'Hospital de la Santa Creu I Sant Pau 08025 Barcelona (ES)**
- **Universitat Autònoma de Barcelona 08193 Barcelona (ES)**

(72) Inventor: **Pol Rigau, Olga**
**08025 Barcelona (ES)**

(74) Representative: **ABG Patentes, S.L.**
**Avenida de Burgos, 16D**
**Edificio Euromor**
**28036 Madrid (ES)**

## (54) Use of heme oxygenase 1 inducers and cannabinoid 2 receptor or delta-opioid receptor agonists in inflammatory pain

(57) The present invention relates to a combination comprising at least an heme oxygenase 1 inducer, and at least a cannabinoid 2 receptor agonist or a selective δ-opioid receptor agonist, for use in the prevention and/or treatment of inflammatory pain.

EP 2 992 880 A1

**Description**

## FIELD OF THE INVENTION

The present invention relates to a combination comprising an heme oxygenase 1 inducer and a cannabinoid 2 receptor agonist or a selective δ-opioid receptor agonist for use in the prevention and/or treatment of inflammatory pain.

## BACKGROUND OF THE INVENTION

The International Association for the Study of Pain defines pain as an unpleasant sensory and emotional experience associated with actual or potential tissue damage, or described in terms of such damage.

Inflammatory pain is a common clinical problem that presents a major challenge to healthcare providers because of its complex natural history, unclear etiology, and poor response to therapy.

It is well known that the administration of μ-opioid receptor (MOR) agonists, δ-opioid receptor (DOR) agonists as well as cannabinoid 2 receptor (CB2R) agonists elicits antiallodynic and antihyperalgesic effects during peripheral inflammation [Obara et al., Pain, 2009, 141, 283-291; Negrete et al., PLoS One, 2011, 6, e26688; Hervera et al., Psychopharmacology, 2013, 228, 463-477].

Opioids, in particular μ-opioid receptor (MOR) agonists, are used in the management of inflammatory pain in humans due to their recognized improved analgesic effects during peripheral inflammation. However, the development of tolerance to their analgesic effects during inflammation is a limiting factor for the utilization of morphine, a MOR agonist, in the management of inflammatory pain, as well as its well-known side effects, such as respiratory depression, nausea, constipation and vomiting.

Selective δ-opioid receptor (DOR) agonists have been described as eliciting an increased antinociceptive potency during inflammatory pain [Leánez et al., Eur J Pharmacol, 2009, 602, 41-49]. Although DOR agonists have shown less potency than morphine in these studies, said compounds have a reduced or absent physical dependence, and reduced respiratory and gastrointestinal impairments.

Cannabinoid 2 receptor (CB2R) agonists have also been described as attenuating inflammatory pain [Negrete et al., PLoS One, 2011, 6, e26688].

Moreover, heme oxygenase 1 (HO-1) inducers have been suggested as being relevant for the treatment of inflammatory pain [Rosa et al., Pain, 2008, 137, 332-339].

It has been reported that cobalt protoporphyrin IX (a HO-1 inducer; CoPP) increases the antinociceptive effect of morphine (a μ-opioid receptor (MOR) agonist) in neuropathic pain and in inflammatory pain [Hervera et al., Anesthesiology, 2013, 118, 1180-1197; Hervera et al., Psychopharmacology, 2013, 228, 463-477] but does not alter the antinociceptive effect of morphine treatment in visceral pain [Hervera et al., Psychopharmacology, 2013, 228, 463-477].

Moreover, it has also been disclosed that cobalt protoporphyrin IX (CoPP) decreased the antinociceptive effect of DPDPE (a DOR agonist) and JWH-015 (a CB2R agonist) in neuropathic pain [Hervera et al., Anesthesiology, 2013, 118, 1180-1197].

As explained above, HO-1 inducers, DOR and CB2R agonists have shown antinociceptive effect when used individually in the treatment of inflammatory pain. However, contrary to the teachings of the prior art, the inventors have surprisingly found that administration of an HO-1 inducer enhances the antinociceptive effects of DOR and CB2R agonists in the treatment of inflammatory pain, i.e. a synergistic effect is obtained by the combination of a HO-1 inducer and a DOR or CB2R agonist, providing thus a new strategy for management of inflammatory pain.

## DESCRIPTION OF THE FIGURES

Figures 1 and 2 show the effects of the subplantar administration of DPDPE or JWH-015 on the mechanical allodynia and thermal hyperalgesia induced by peripheral inflammation. Mechanical antiallodynic and thermal antihyperalgesic effects produced by the subplantar administration of different doses DPDPE (Fig. 1A and 1B) or JWH-015 (Fig. 2A and 2B) and their respective vehicles in the ipsilateral paw of CFA-injected mice. Data are expressed as mean values of maximal possible effect (%) ± SEM (6 animals for dose). For each test, drug and dose, * denotes significant differences versus their respective vehicle treated animals ($P < 0.05$; one way ANOVA followed by the Student Newman Keuls test).

Figure 3 shows the effects of CoPP on the antiallodynic and antihyperalgesic responses to DPDPE. Mechanical antiallodynic (A) and thermal antihyperalgesic (B) effects produced by the subplantar administration of 50 μg of DPDPE or vehicle in the ipsilateral paw of CFA-injected mice pretreated with 10 mg/kg of CoPP. The effect of the intraperitoneal administration of CoPP alone is also shown. Data are expressed as mean values of the maximal

possible effect (%) ± SEM (6 animals per group). For each behavioral test, * denotes significant differences versus control group treated with vehicle ($P < 0.05$, one way ANOVA followed by Student Newman Keuls test), + denotes significant differences versus control group treated with DPDPE ($P < 0.05$, one way ANOVA followed by the Student Newman Keuls test) and # denotes significant differences versus group treated with CoPP plus vehicle ($P < 0.05$; one way ANOVA followed by the Student Newman Keuls test).

Figure 4 shows the effects of CoPP on the antiallodynic and antihyperalgesic responses to JWH-015. Mechanical antiallodynic (A) and thermal antihyperalgesic (B) effects produced by the subplantar administration of 30 μg of JWH-015 or vehicle in the ipsilateral paw of CFA-injected mice pretreated with 10 mg/kg of CoPP. The effect of the intraperitoneal administration of CoPP alone is also shown. Data are expressed as mean values of the maximal possible effect (%) ± SEM (6 animals per group). For each behavioral test, * denotes significant differences versus control group treated with vehicle ($P < 0.05$, one way ANOVA followed by Student Newman Keuls test), + denotes significant differences versus control group treated with JWH-015 ($P < 0.05$, one way ANOVA followed by the Student Newman Keuls test) and # denotes significant differences versus group treated with CoPP plus vehicle ($P < 0.05$; one way ANOVA followed by the Student Newman Keuls test).

Figure 5 shows the effects of tin protoporphyrin IX (SnPP) treatment on the antiallodynic and antihyperalgesic responses to DPDPE or JWH-015. Mechanical antiallodynic (Fig. 5A) and thermal antihyperalgesic (Fig. 5B) effects of the subplantar administration of DPDPE (150 μg) or JWH-015 (300 μg) combined with SnPP (290 μg) in the ipsilateral paw of CFA-injected mice are shown. The effects of the subplantar administration of DPDPE, JWH-015, SnPP or vehicle alone are also represented. Data are expressed as mean values of the maximal possible effect (%) ± SEM (6 animals per group). For each behavioral test, * denotes significant differences versus control group treated with vehicle ($P < 0.05$, one way ANOVA followed by Student Newman Keuls test), + denotes significant differences versus control group treated with SnPP ($P <0.05$, one way ANOVA followed by the Student Newman Keuls test) and # denotes significant differences versus group treated with DPDPE or JWH-015 plus vehicle ($P < 0.05$, one way ANOVA followed by the Student Newman Keuls test).

Figures 6-8 show the effect of CoPP on HO-1, DOR and CB2R protein expression from CFA-injected mice. The protein expression of HO-1 (Fig. 6), DOR (Fig. 7) and CB2R (Fig. 8) in the ipsilateral site of the dorsal root ganglia from CFA-injected mice treated with vehicle (CFA-vehicle) or CoPP (CFA-CoPP) are represented. The expression of this enzyme and receptors in the dorsal root ganglia from naive mice treated with vehicle (naive-vehicle) has been also represented as controls. For each protein, * indicates significant differences when compared versus naive vehicle treated mice ($P < 0.05$, one-way ANOVA followed by Student-Newman-Keuls test). Representative examples of Western blots for HO-1, DOR and CB2R proteins, in which β-actin was used as a loading control, are also shown. Data are expressed as mean values ± SEM; n = 5 samples per group.

## DESCRIPTION OF THE INVENTION

The present invention relates to a combination comprising:

(a) at least an heme oxygenase 1 inducer, and
(b) at least a cannabinoid 2 receptor agonist or a selective δ-opioid receptor agonist,

for use in the prevention and/or treatment of inflammatory pain.

The term "heme oxygenase" or "HO", as used herein, refers to an enzyme which catalyzes the degradation of the heme group. This degradation produces biliverdin, iron and carbon monoxide. There are three known isoforms of heme oxygenase; heme oxygenase 1 or HO-1, heme oxygenase 2 or HO-2, and heme oxygenase 3 or HO-3. HO-1 is an isoform inducible in response to stress such as oxidative stress, hypoxia, heavy metals, and cytokines, among others. HO-2 is a constitutive isoform that is expressed under homeostatic conditions. Both HO-1 and HO-2 are ubiquitously expressed and catalytically active. HO-3 is not catalytically active. HO-1 is responsible for regulating cellular levels of heme, for the recycling of iron from senescent red blood cells and extrahematopoietic cells, such as liver cells. In human, HO-1 is encoded by the gene HMOX1, identified in the Genebank database by the Gene ID: 3162.

The term "heme oxygenase inducer", as used herein, refers to any molecule that increases the activity of HO-1, and thus increases the degradation of heme to produce biliverdin, iron and carbon monoxide.

The person skilled in the art knows how to determine the ability of a particular molecule for increasing HO-1 activity and thus, to determine if this particular molecule is a HO-1 inducer. For example, the ability of a molecule for increasing HO-1 activity can be determined using the methodology described by Benallaoua et al., Arthritis Rheum. 2007,56, 2585-2594). Briefly, HO-1 activity in protein extracts from spinal cord, dorsal root ganglia or paw tissues from rodents treated with 5, 10, 25 or 50 mg/kg of the test compound after 6 to 24 hours of its intraperitoneal administration may be evaluated spectrophotometrically, using hemin (as substrate) and excess biliverdin reductase. Briefly, tissues are first rinsed in ice-cold 1.15% KCl-20 mM Tris HCl buffer (pH 7.4). The homogenate is then centrifuged at 5,000g for

20 minutes at 4°C and then at 105,000 g for 1 hour at 4°C. The supernatant, which contains microsomal biliverdin reductase, is incubated for 30 minutes in the dark at 37°C, in a reaction mixture containing hemin, NADPH, G6P, G6PDH, and tissue protein extracts. Bilirubin production is measured spectrophotometrically and expressed as picomoles of bilirubin per milligram of protein per hour. The HO-1 activity corresponds to said bilirubin production.

In some embodiments, induction refers to an increase of 10% or more, 15% or more, 20% or more, 25% or more, 30% or more, 40% or more, 50% or more, 60% or more, 70% or more, 80% or more or 90% or more in comparison to the basal activity of the enzyme.

Illustrative non-limitative examples of HO-1 inducers are $SnCl_2$, $CoCl_2$, hemin, heme arginate, iron protoporphyrin IX, cobalt protoporphyrin IX (also known as CoPP or cobalt (III) protoporphyrin IX chloride), vitamin B12, 1,2-bis(nicotinamido)propane (disclosed in EP1266660A1, which is incorporated herein by reference), and the compounds 1-methyl-2-(6-trifluoromethoxy-benzothiazol-2-ylamino)-1H-benzoimidazole-5-carboxylic acid (2-methoxy-ethyl)-amide, 1-methyl-2-(6-trifluoromethoxy-benzothiazo 1-2-ylamino)-1 H-benzoimidazo le-5-carboxylic acid dimethylcarbamoylmethyl-amide, 1 -methyl-2-(6-trifluoromethoxy-benzothiazo 1-2-ylamino)-1H-benzoimidazole-5-carboxylic acid [2-(2-hydroxy-ethoxy)-ethyl)]-amide and 2-(benzothiazol-2-ylamino)-1H-benzoimidazole-5-carboxylic acid (1H-indazol-6-yl)-amide (disclosed in WO 2012/094580 which is incorporated herein by reference).

Table 1. Structure of some HO-1 inducers.

| Name | Structure |
|---|---|
| hemin | 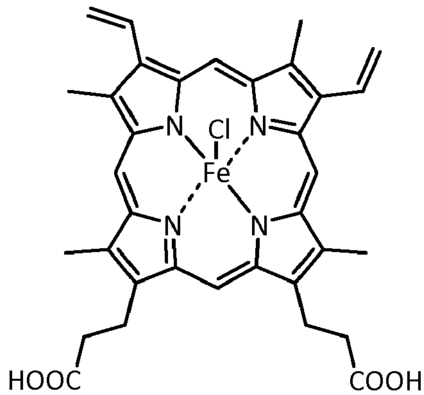 |
| heme arginate | 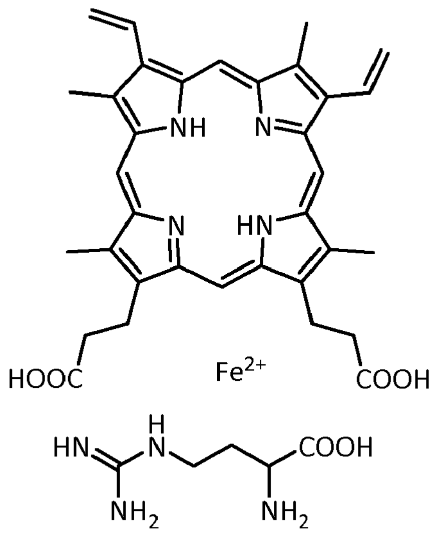 |

(continued)

| Name | Structure |
|---|---|
| iron protoporphyrin IX | 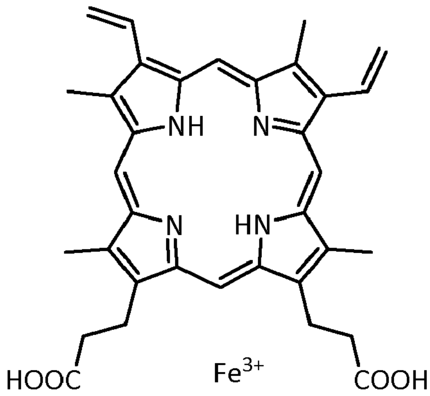 |
| CoPP | 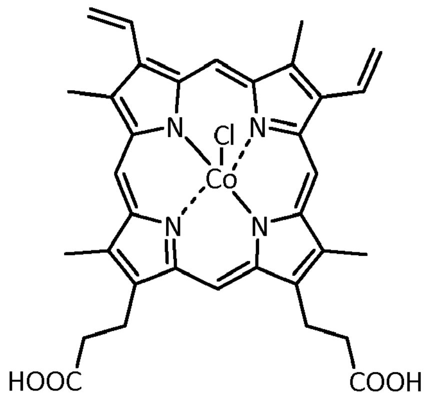 |
| 1,2-bis(nicotinamide)propane | 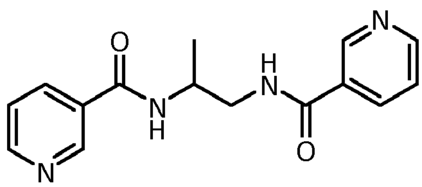 |
| 1-methyl-2-(6-trifluoromethoxy-benzo thiazol-2-yl amino)-1H-benzo imidazole-5-carboxylic acid (2-methoxy-ethyl)-amide | 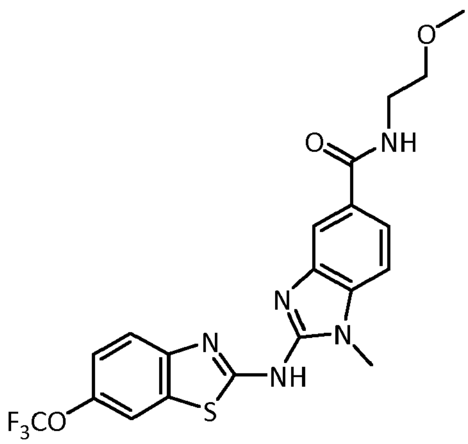 |

(continued)

| Name | Structure |
| --- | --- |
| 1-methyl-2-(6-trifluoromethoxy-benzo thiazol-2-yl amino)-1H-benzo imidazole-5-carboxylic acid dimethyl carbamoylmethyl-amide | 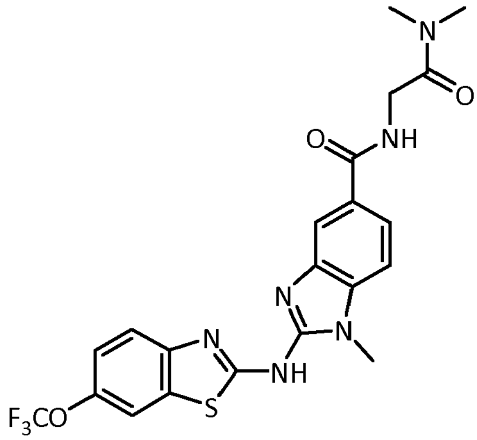 |
| 1-methyl-2-(6-trifluoromethoxy-benzo thiazol-2-ylamino)-1H-benzo imidazole-5-carboxylic acid [2-(2-hydroxy-ethoxy)-ethyl)]-amide | 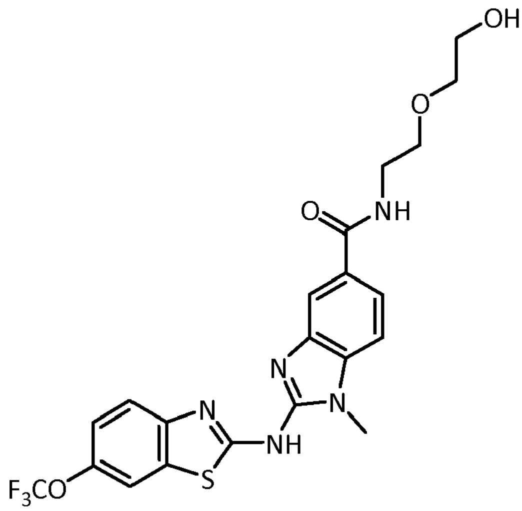 |
| 2-(benzothiazol-2-ylamino)-1H-benzo imidazole-5-carboxylic acid (1H-indazol-6-yl)-amide | 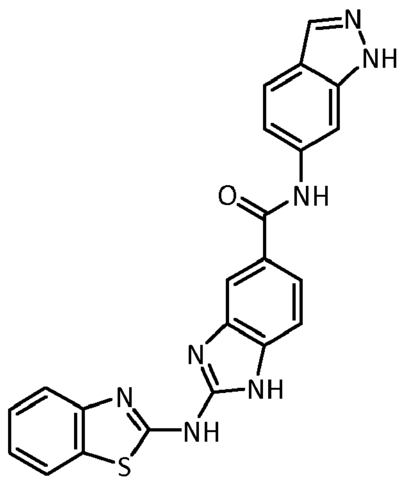 |

In a preferred embodiment, the HO-1 inducer is selected from the group consisting of $SnCl_2$, $CoCl_2$, hemin, heme arginate, iron protoporphyrin IX, cobalt protoporphyrin IX (CoPP), vitamin B12, 1,2-bis(nicotinamido)propane, 1-methyl-2-(6-trifluoromethoxy-benzothiazol-2-ylamino)-1H-benzoimidazole-5-carboxylic acid (2-methoxy-ethyl)-amide, 1-methyl-2-(6-trifluoromethoxy-benzothiazol-2-ylamino)-1H-benzoimidazole-5-carboxylic acid dimethylcarbamoylmethyl-amide, 1-methyl-2-(6-trifluoromethoxy-benzothiazol-2-ylamino)-1H-benzoimidazole-5-carboxylic acid [2-(2-hydroxy-ethoxy)-ethyl)]-amide and 2-(benzothiazol-2-ylamino)-1H-benzoimidazole-5-carboxylic acid (1H-indazol-6-yl)-amide or a pharmaceutically acceptable salt thereof. Preferably, the HO-1 inducer is CoPP or a pharmaceutically acceptable salt thereof

The term "δ-opioid receptor", "delta receptor" or "DOR", as used herein, refers to a opioid receptor, which is a G protein-coupled receptor, that has enkephalins as its endogenous ligands. This receptor is localized in the brain (pontine nuclei, amygdala, olfactory bulbs and deep cortex) and peripheral sensory neurons. In humans the δ-opioid receptor is encoded by the gene OPRD1, identified in the Genebank database by the Gene ID: 4985.

The term "δ-opioid receptor agonist", as used herein, refers to any molecule that binds to the δ-opioid receptor and activates the receptor to produce a biological response.

The person skilled in the art knows how to determine the affinity of a particular molecule for the δ-opioid receptor and thus, to determine if this particular molecule is an agonist of the δ-opioid receptor. For example, the δ-opioid receptor affinity of a molecule can be determined using the methodology described by Quock et al. [European Journal of Pharmacology, 1997, 326, 101-104]. Briefly, membranes from CHO cells that express the δ-opioid receptor are incubated with appropriate concentrations of the compound to be tested in the presence of 0.5 nM [3H]naltrindole (32 mmol/Ci, New England Nuclear) in a total assay buffer of 1.0 ml. After 90 min incubation at 30°C, the reaction is terminated by rapid filtration under vacuum through Whatman GF/B glass fiber filters, followed by four washes with 4 ml ice-cold 25 mM Tris/120 mM NaCl, pH 7.4. Bound radioactivity is measured by liquid scintillation spectrophotometry after an overnight extraction with EcoLite scintillation cocktail (ICN Biomedicals, Costa Mesa, CA, USA). Nonspecific binding is determined using 10 μM naltrexone.

Data from the [3H]naltrindole binding assay are analyzed using Graph Pad Prism (San Diego, CA, USA). The Ki value is determined from [3H]naltrindole competitive inhibition experiments using the Cheng-Prusoff equation (Cheng and Prusoff, 1973, Biochem Pharmacol. 1973, 22:3099-3108).

The term "selective δ-opioid receptor agonist", as used herein, refers to an agonist of the δ-opioid receptor that is selective for the δ-opioid receptor with respect to the μ-opioid receptor and has a $K_i$ for the δ-opioid receptor which is one fourth or less than that of the μ-opioid receptor or, more preferably, is one tenth or less than the $K_i$ for the μ-opioid receptor, or even more preferably, a $K_i$ with respect to the δ-opioid receptor which is one hundredth or less that for the μ-opioid receptor.

To determine if this particular molecule could be an agonist of the μ-opioid receptor, for example, the μ-opioid receptor affinity of a molecule can be determined using the methodology described by Wang et al. [Neuroscience Letters 2008, 443, 209-212]. Briefly, binding assay with [3H] naloxone may be performed with membranes from CHO cells transfected with μ-opioid receptors. Indeed, the CHO cells transfected with μ-opioid receptors are homogenized in 10 mL Tris-HCl buffer (pH 7.4). The homogenate is centrifuged at 2850 g for 10 min. The supernatant is then collected and centrifuged at 48,000 g for 20 min. The pellet that contained cell membranes is homogenized in Tris-HCl buffer. The membrane fraction (0.5 mL) is incubated with $10^{12}$ to $10^{-3}$M of the particular molecule or $10^{-10}$ to $10^{-7}$ M of naloxonazine, for 30 min. The radioligand [3H] naloxone (14.5 Ci), is then added and the reaction is allowed to proceed for 30 min until terminated with ice. The solution is filtered through a Whatman GF/B filter that is washed three times with 2 mL ice-cold water. The filter is placed into a scintillation vial with 10mL of scintillation liquid. The radioactivity (cpm) was measured with a counter (Beckman, LS5000TA). The specific binding is calculated by subtracting the nonspecific binding measured by standard solutions from the total binding. Data from the [3H] naltrindole binding assay are analyzed using Graph Pad Prism (San Diego, CA, USA). The Ki value is determined from [3H]naltrindole competitive inhibition experiments using the Cheng-Prusoff equation (Cheng and Prusoff, 1973, Biochem Pharmacol. 1973, 22:3099-3108).

In some embodiments, the selective δ-opioid receptor from 1 μM to 0.01 nM, preferably from 500 nM to 1 nM, more preferably from 250 nM to 1 nM, still more preferably from 100 nM to 1 nM.

Illustrative non-limitative examples of selective δ-opioid receptor agonist are DPDPE ([D-Pen2,5]enkephalin, where Pen is penicillamine), dermenkephalin (D-$Ser^2$, $Leu^5$, $Thr^6$]-enkephalin), deltorphin I, deltorphin II, SNC-80, (+)-BW373U86, DPI-287, DPI-221, TAN-67, RWJ-394674, 7-spiroindanyloxymorphone, DSLET and SNC-162, whose structure is shown in Table 2.

Table 2. Structure of DOR agonists.

| Name | Structure |
|---|---|
| DPDPE | Tyr-D-Pen-Gly-Phe-D-Pen |
| dermenkephalin | Tyr-D-Met-Phe-His-Leu-Met-Asp-$NH_2$ |
| deltorphin I | Tyr-D-Ala-Phe-Asp-Val-Val-Gly-$NH_2$ |
| deltorphin II | Tyr-D-Ala-Phe-Glu-Val-Val-Gly-$NH_2$ |

(continued)

| Name | Structure |
|---|---|
| SNC-80 | 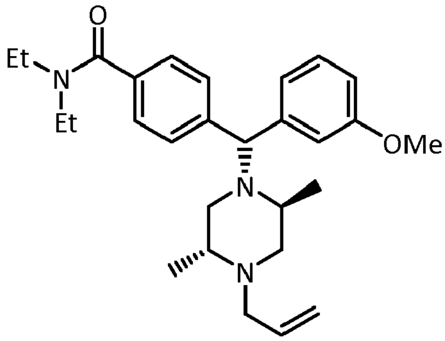 |
| (+)-BW373U86 | 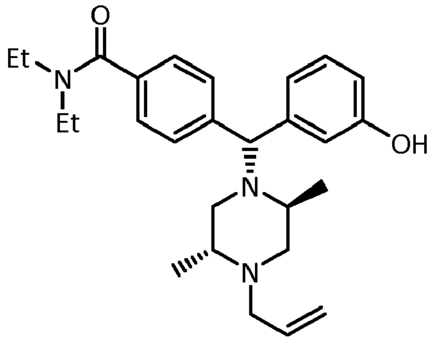 |
| DPI-287 | 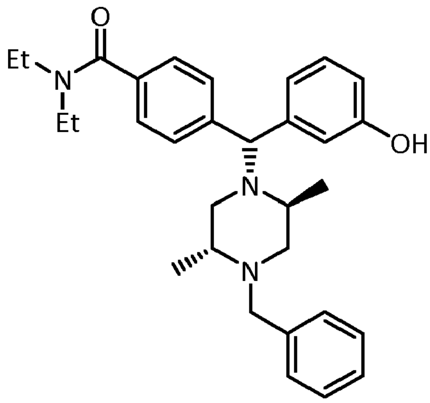 |
| DPI-221 | 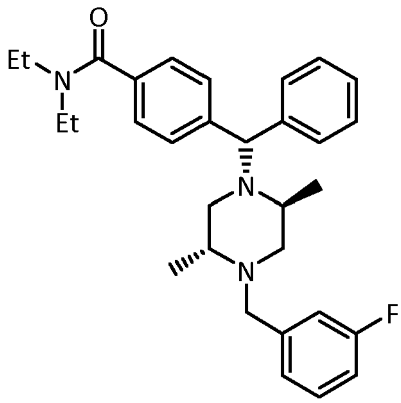 |

(continued)

| Name | Structure |
|---|---|
| TAN-67 | 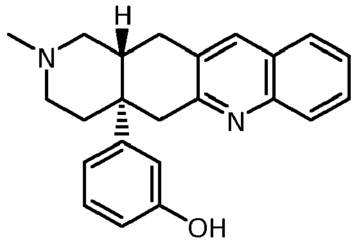 |
| RWJ-394674 | 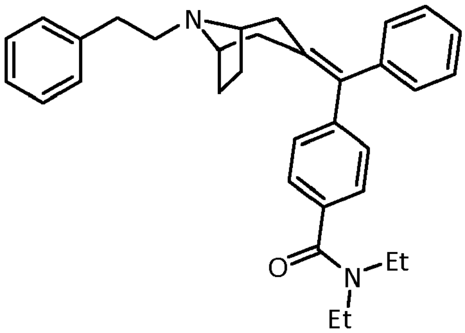 |
| 7-spiroindanyloxymorphone | 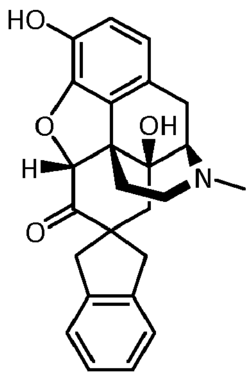 |
| DSLET | Tyr-D-Ser-Gly-Phe-Leu-Thr |
| SNC-162 | 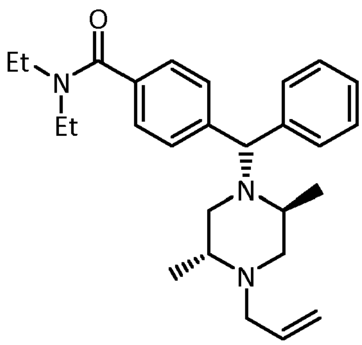 |

In a preferred embodiment, the selective δ-opioid receptor agonist is selected from the group consisting of DPDPE ([D-pen2,5]enkephalin, where pen is penicillamine), dermenkephalin, deltorphin I, deltorphin II, SNC-80, (+)-BW373U86, DPI-287, DPI-221, TAN-67, RWJ-394674, 7-spiroindanyloxymorphone, DSLET and SNC-162, or pharmaceutically acceptable salts thereof. Preferably the selective δ-opioid receptor agonist is DPDPE or a pharmaceutically acceptable salt thereof.

The term "cannabinoid 2 receptor", "CB2 receptor" or "CB2R", as used herein, refers to a member of the family of the cannabinoid receptors, which are G protein-coupled receptors that are activated by cannabinoids. The cannabinoid receptor CB2 is expressed in T cells of the immune system, on macrophages, B cells, and hematopoitic cells, but it is

also expressed in peripheral nerve terminals. In humans the cannabinoid receptor CB2 is encoded by the gene CNR2, identified in the Genebank database by the Gene ID: 1269.

The term "cannabinoid 2 receptor agonist", as used herein, refers to any molecule that binds to the cannabinoid 2 receptor and activates the receptor to produce a biological response.

The person skilled in the art knows how to determine the affinity of a particular molecule for the cannabinoid 2 receptor and thus, to determine if this particular molecule is an agonist of the cannabinoid 2 receptor. For example, the cannabinoid 2 receptor affinity of a molecule can be determined using the methodology described by Thomas et al. [Br. J. Pharmacol. 2007, 150, 613-623]. Briefly, binding assays with [3H]CP55,940 may be performed with membranes from CHO cells transfected with CB2 receptors. The assays are carried out with [3H]CP55,940, 1 mg/ml bovine serum albumin (BSA) and 50 mM Tris buffer, total assay volume 500 ml, using the filtration procedure described previously (Thomas et al., Br J Pharmacol, 2005, 146, 917-926). Binding is initiated by the addition of either the tissue or cell membranes (33 mg protein per tube) or the CB2-CHO cells (25 mg protein per tube) and all assays are performed at 37°C for 60 min. Specific binding is defined as the difference between the binding that occurrs in the presence and the absence of 1 mM unlabelled CP55,940. The concentration of [3H]CP55,940 used in the displacement assays is 0.7 nM. All drugs are stored as a stock solution of 10 mM in dimethyl sulphoxide (DMSO), the vehicle concentration in all assay tubes being 0.1% DMSO. The binding parameters for [3H]CP55,940 are determined by fitting data from saturation-binding experiments to a one-site saturation plot by using GraphPad Prism. The dissociation constant (Ki) is calculated using the equation of Cheng and Prusoff 1973 (Biochem Pharmacol. 22:3099-31089).

In some embodiments, the cannabinoid 2 receptor agonist has a $K_i$ from 1 μM to 1 nM, preferably from 500 nM to 1 nM, more preferably from 100 nM to 1 nM, still more preferably from 50 nM to 1 nM, even more preferably from 25 nM to 1 nM. Preferably, such a cannabinoid agonist is selective for the CB2 receptor and has a $K_i$ for the CB2 receptor which is one fourth or less than that of the CB1 receptor or, more preferably, is one tenth or less than the $K_i$ for the CB1 receptor, or even more preferably, a $K_i$ with respect to the CB2 receptor which is one twenty-fifth or less that for the CB1 receptor.

To determine the Ki of a particular molecule with respect to the cannabinoid 1 receptor, the methodology described by Leggett et al. [Br. J. Pharmacol. 2004, 141, 253-262] can be used. Briefly, binding assays with [3H]CP55,940 may be performed with membranes from HEK-293T cells transfected with CB1 receptors, as previously described by Abadji et al., (J Neurochem., 1999, 72:2032-2038). Membrane preparations are incubated with 4 nM [3H]CP55,940 with varying concentrations of the molecule to test. Nonspecific binding is determined with 1 μM CP55,940. All conditions are prepared in triplicate. A Brandel cell harvester (Brandel Inc., Gaitherburg, MD, U.S.A.) is used to separate bound from unbound ligand and the former quantified by liquid scintillation counting. Results are analysed with Prism GraphPAD Software (GraphPAD Software, San Diego, CA, U.S.A.) using the Cheng-Prusoff 1973 (Biochem Pharmacol. 22:3099-31089) equation to determine the dissociation constant (Ki) with the KD value of 4.7 nM for CP55,940 binding to CB1 HEK 293T membrane preparations.

Illustrative non-limitative examples of cannabinoid 2 receptor agonists include JWH-015, JWH-133, AM-1241, HU-308, GW 405833, L-759,633, L-759,656, CB65, GP1a, GP2a, MDA 19, SER 601 whose structure is shown in Table 3.

Table 3. Structure of CB2R agonists.

| Name | Structure |
|---|---|
| JWH-015 | 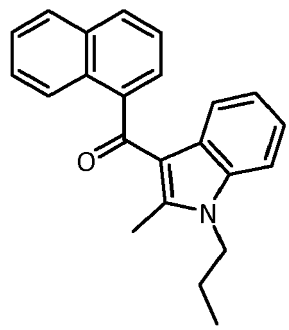 |

(continued)

| Name | Structure |
|---|---|
| JWH-133 | 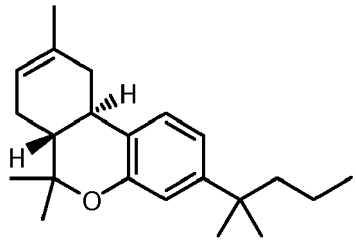 |
| AM-1241 | 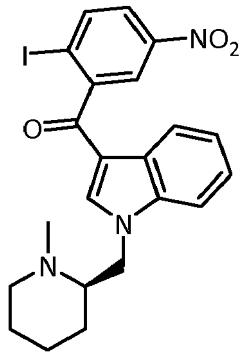 |
| HU-308 | 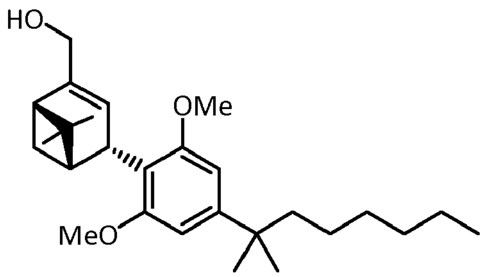 |
| GW 405833 | 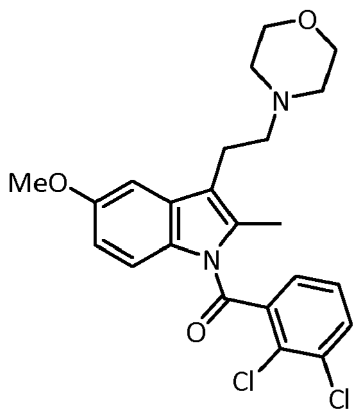 |
| L-759,633 | 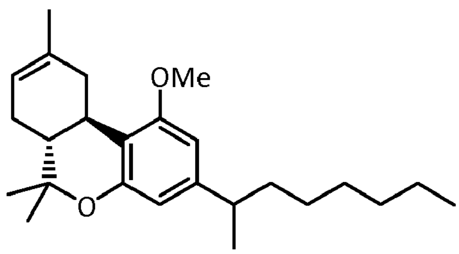 |

(continued)

| Name | Structure |
|---|---|
| L-759,656 | 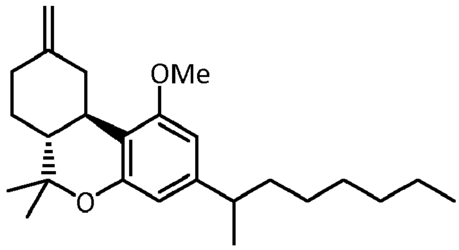 |
| CB65 | 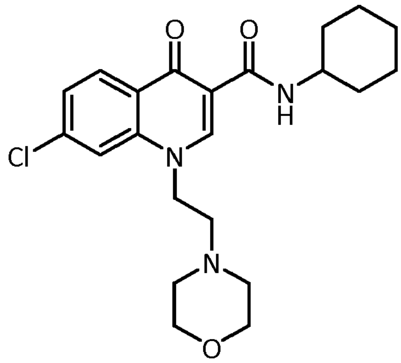 |
| GP1a | 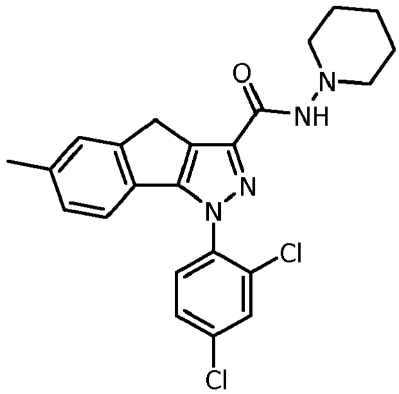 |
| GP2a | 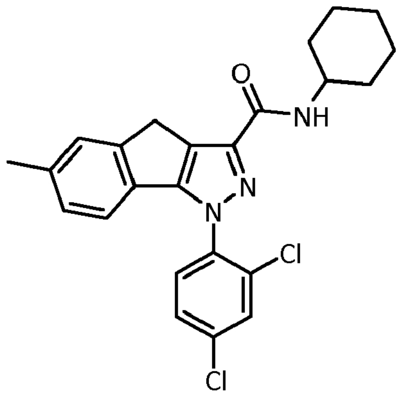 |

(continued)

| Name | Structure |
|---|---|
| MDA 19 | 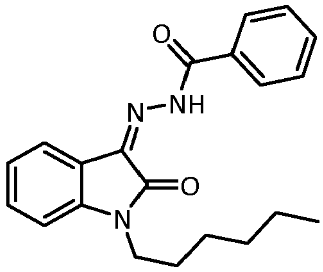 |
| SER 601 | 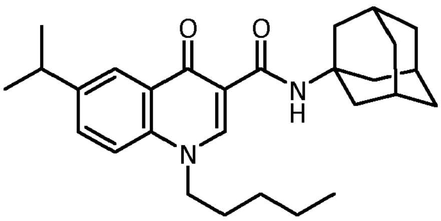 |

In a preferred embodiment, the cannabinoid 2 receptor agonist is selected from the group consisting of JWH-015, JWH-133, AM-1241, HU-308, GW 405833, L-759,633, L-759,656, CB65, GP1a, GP2a, MDA 19 and SER 601, or pharmaceutically acceptable salts thereof, preferably JWH-015 or a pharmaceutically acceptable salt thereof.

In a particular embodiment, the combination of the present invention comprises CoPP or a pharmaceutically acceptable salt thereof, as the HO-1 inducer, and DPDPE or a pharmaceutically acceptable salt thereof, as the DOR agonist.

In another particular embodiment, the combination of the present invention comprises CoPP or a pharmaceutically acceptable salt thereof, as the HO-1 inducer, and JWH-015 or a pharmaceutically acceptable salt thereof, as the CB2R agonist.

In a preferred embodiment, the ratio by weight of (a) the heme oxygenase 1 inducer and (b) the cannabinoid 2 receptor agonist or selective δ-opioid receptor agonist is from 1:0.01 to 1:2 preferably from 1:0.1 to 1:0.2.

As used herein, the term "pharmaceutically acceptable salt" embraces salts with a pharmaceutically acceptable acid or base, which are synthesized from the parent compound which contains an acidic moiety by addition of a pharmaceutically acceptable base, or which are synthesized from the parent compound which contains a basic moiety by addition of a pharmaceutically acceptable acid. Pharmaceutically acceptable acids include both inorganic acids, for example, hydrochloric, sulfuric, phosphoric, diphosphoric, hydrobromic, hydroiodic, and nitric acid, and organic acids, for example, citric, fumaric, maleic, malic, mandelic, ascorbic, oxalic, succinic, tartaric, benzoic, acetic, methanesulfonic (mesylate), ethanesulfonic, benzenesulfonic (besylate), or p-toluenesulfonic (tosylate) acid. Pharmaceutically acceptable bases include alkali metal (e.g., sodium or potassium) and alkali earth metal (e.g., calcium or magnesium) hydroxides and organic bases, such as alkyl amines, arylalkyl amines, and heterocyclic amines. For instance, pharmaceutically acceptable salts of compounds provided herein are synthesized from the parent compound which contains a basic or an acid moiety by conventional chemical methods. Generally, such salts are, for example, prepared by reacting the free base or free acid forms of these compounds with a stoichiometric amount of the appropriate acid or base, respectively, in water or in an organic solvent or in a mixture of the two. The term "pharmaceutically acceptable salt" also embraces coordination of the metal present in the protoporphyrin structure with a ligand such as fluoro, chloro, bromo, and iodo, preferably chloro.

The term "treatment" and "treating" in the context of this specification mean administration of a compound or formulation of the invention to eliminate or ameliorate inflammatory pain, or symptoms associated to inflammatory pain.

The terms "prevention" and "preventing" in the context of this specification mean inhibiting the onset of inflammatory pain, or symptoms associated to inflammatory pain or reducing their intensity.

In the context of the present invention, "inflammatory pain" is to be understood as the result of an inflammatory response to tissue damage, such as pinched nerves, surgical procedures, cancer, arthritis and as a result of an acute (e.g. stroke) and chronic (e.g. alzeheimer's disease, degenerative disease and multiple sclerosis) neurodegenerative disorder; and as a result of a development of diabetes, autoimmune, cardiovascular, gastrointestinal or pulmonary diseases.

Most patients with inflammatory pain do not experience pain continuously but they experience more pain when

they move the inflamed site. Symptoms and signs arising from normal tissues exposed to high intensity stimuli generally reflect the intensity, localization and timing of the initiating stimuli. In contrast, pain arising from inflamed or injured tissues may arise spontaneously in the absence of an external trigger. Alternatively, responses to noxious stimuli may be enhanced (hyperalgesia) or normally innocuous stimuli may produce pain (allodynia).

In a particular embodiment, the combination defined herein is for use in the treatment and/or prevention of chronic inflammatory pain. For the purposes of the present invention, "chronic inflammatory pain" is considered any inflammatory pain that persists longer than the reasonable expected healing time for the involved tissues. In an embodiment of the invention it is considered ongoing inflammatory pain lasting longer than 1 month, preferably longer than 2 months. In a further embodiment of the invention, "chronic inflammatory pain" is considered any pain that persists longer than 3 months, preferably longer than 6 months.

The combination defined above may be formulated for its simultaneous, separate or sequential administration. This has the implication that the combination of HO-1 inducer and the DOR or CB2R agonist may be administered:

a) As a combination that is being part of the same medicament composition, both being then administered always simultaneously.

b) As a combination of two compositions, a first composition comprising the HO-1 inducer and a second composition comprising the DOR or CB2R agonist enabling the possibility of simultaneous, sequential or separate administration. In a particular embodiment, the two compositions are independently administered at the same time. In another particular embodiment, the HO-1 inducer is administered first, and then the DOR or CB2R agonist is separately or sequentially administered. In yet another particular embodiment, the DOR or CB2R agonist is administered first, and then the HO-1 inducer is administered, separately or sequentially.

In general, the HO-1 inducer, and the DOR or CB2R agonist, either as being part of the same composition or as two different compositions, should be administered in association with one or more pharmaceutically acceptable excipients.

The term "pharmaceutically suitable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

The compositions of the present invention may be administered orally, parenterally, subcutaneously, rectally, topically, by inhalation, and locally. Any administration method commonly used for drugs, such as tablets, coated tablets, capsules, solutions, suspensions, syrups, powders, suppositories, creams, and ointments, may be used. The pharmaceutical composition can be formulated employing conventional liquid or solid vehicles or diluents and pharmaceutical additives, according to the desired mode of administration. The mentioned formulations will be prepared using standard methods, such as those described or referred to in the Spanish and U.S. Pharmacopoeias and similar reference texts.

The DOR or CB2R agonist comprised in the combination defined herein is preferably administered locally, i.e. at the site where the patient experiences the inflammatory pain. The administration of HO-1 inductor is preferably systemic, i.e. by subcutaneous or intraperitoneal administration.

A further embodiment of the present invention is the use of a combination as defined above for use in the manufacture of a medicament for the treatment of inflammatory pain, preferably chronic inflammatory pain.

Still a further embodiment of the present invention is a method for the treatment of inflammatory pain, preferably chronic inflammatory pain, wherein a combination as defined above is administered to a patient in need thereof.

## Examples

### Materials and Methods

Experimental animals

The experiments were performed in male C57BL/6 mice acquired from Harlan Laboratories (Barcelona, Spain). All mice weighing 21 to 25 g were housed under 12-h/12-h light/ dark conditions in a room with controlled temperature (22° C) and humidity (66%). Animals had free access to food and water and were used after a minimum of 7 days acclimatization to the housing conditions. All experiments were performed according with the Guide for the Care and Use of Laboratory Animals as adopted and promulgated by the U.S. National Institutes of Health and approved by the local Committee of Animal Use and Care of the Autonomous University of Barcelona. All efforts were made to minimize animal suffering and to reduce the number of animals used.

Induction of chronic inflammatory pain

Chronic inflammatory pain was induced by the subplantar injection of 30 μl of CFA (Sigma) into the right hind paw under brief anesthetic conditions with isoflurane according to previous works (Leánez et al., Eur J Pharmacol, 2009, 602:41-49; Hervera et al., Naunyn Schmiedebergs Arch Pharmacol, 2009, 380:345-352). All experiments were performed at 10 days after CFA injection. At this time point, all of these animals developed a local inflammatory reaction, allodynia to mechanical stimuli and hyperalgesia to noxious thermal stimuli, as previously reported (Negrete et al., PLoS One, 2011, 6:e26688). The development of mechanical allodynia and thermal hyperalgesia was evaluated by using the von Frey filaments and plantar tests, respectively. All animals were tested in each paradigm before and at 10 days after CFA-injection.

Nociceptive behavioral tests.

*Mechanical allodynia* was quantified by measuring the hind paw withdrawal response to von Frey filament stimulation. In brief, animals were placed in methacrylate cylinders (20 cm high, 9 cm diameter; Servei Estació, Barcelona, Spain) with a wire grid bottom through which the von Frey filaments (North Coast Medical, Inc., San Jose, CA) with a bending force in the range of 0.008-3.5 g were applied by using a modified version of the up-down paradigm, as previously reported by Chaplan et al. (J Neurosci Methods, 1994, 53:55-63). The filament of 0.4 g was used first and the 3.0 g filament was used as a cut-off. Then, the strength of the next filament was decreased or increased according to the response. The threshold of response was calculated from the sequence of filament strength used during the up-down procedure by using an Excel program (Microsoft Iberia SRL, Barcelona, Spain) that includes curve fitting of the data. Clear paw withdrawal, shaking, or licking of the paw was considered as a nociceptive-like response. Both ipsilateral and contralateral hind paws were tested. Animals were allowed to habituate for 1 h before testing in order to allow an appropriate behavioral immobility.

*Thermal hyperalgesia* was assessed as previously reported by Hargreaves et al. (Pain, 1988, 32:77-88). Paw withdrawal latency in response to radiant heat was measured using the plantar test apparatus (Ugo Basile, Varese, Italy). Briefly, mice were placed in methacrylate cylinders (20 cm high x 9 cm diameter) positioned on a glass surface. The heat source was positioned under the plantar surface of the hind paw and activated with a light beam intensity, chosen in preliminary studies to give baseline latencies from 8 to 10 s in control mice. A cut-off time of 12 s was used to prevent tissue damage in absence of response. The mean paw withdrawal latencies from the ipsilateral and contralateral hind paws were determined from the average of three separate trials, taken at 5 min intervals to prevent thermal sensitization and behavioral disturbances. Animals were habituated to the environment for 1 h before the experiment to become quiet and to allow testing.

Western blot analysis

Animals were sacrificed at 0 days (naive) and after CFA-injection by cervical dislocation. Tissues from the ipsilateral section of the dorsal root ganglia (L3 to L5) were removed immediately after killing, frozen in liquid nitrogen, and stored at -80°C until assay. Samples from five animals were pooled into one experimental sample to obtain enough protein levels for performing Western blot analysis. The HO-1, DOR and CB2R protein levels were analyzed by Western blot. Tissues were homogenized in ice-cold lysis buffer (50 mM Tris·Base, 150 nM NaCl, 1% NP-40, 2 mM EDTA, 1 mM phenylmethylsulfonyl fluoride, 0.5 Triton X-100, 0.1% sodium dodecyl sulfate, 1 mM $Na_3VO_4$, 25 mM NaF, 0.5 % protease inhibitor cocktail, and 1% phosphatase inhibitor cocktail). All reagents were purchased at Sigma (St. Louis, MO) with the exception of NP-40 from Calbiochem (Darmstadt, Germany). The crude homogenate was solubilised for 1 h at 4°C, sonicated for 10 s and centrifugated at 4°C or 15 min at 700 g. The supernatant (60 μg of total protein) was mixed with 4 x laemmli loading buffer and then loaded onto 4% stacking/10% separating sodium dodecyl sulfate polyacrylamide gels.

The proteins were electrophoretically transferred onto PVDF membrane for 120 min, blocked with PBS + 5% nonfat dry milk, and subsequently incubated overnight at 4°C with polyclonal rabbit anti-HO-1 (1:300, Stressgen, Ann Arbor, MI), anti-DOR (1:2500, Chemicon-Millipore) or anti-CB2R (1:500, Abcam, Cambridge, United Kingdom). The proteins were detected by a horseradish peroxidase-conjugated anti-rabbit secondary antibody (GE Healthcare, Little Chalfont, Buckinghamshire, United Kingdom) and visualized with chemiluminescence reagents (ECL kit; GE Healthcare) and by exposure onto hyperfilm (GE Healthcare). The intensity of blots was quantified by densitometry. The membranes were stripped and reproved with a monoclonal rabbit anti-β-actin antibody (1:10.000, Sigma) used as a loading control.

Experimental procedure

In a first set of experiments, we assessed the expression of inflammatory pain induced by the subplantar administration of CFA as previously used (Leánez et al., Eur J Pharmacol, 2009, 602:41-49). After the habituation period,

baseline responses were established in the following sequence: von Frey filaments and plantar test. After baseline measurements, inflammatory pain was induced and animals were again tested in each paradigm at day 10 after CFA injection by using the same sequence as for baseline responses. The contralateral paws were used as controls (n = 6 animals per group).

In a second set of experiments, we evaluated the mechanical antiallodynic and thermal antihyperalgesic effects of the subplantar administration of different doses of a specific DOR (DPDPE) or CB2R (JWH-015) agonist and their respective vehicles in the contralateral and ipsilateral paw of CFA-injected animals (n = 6 animals per group).

In a third set of experiments, we investigated the mechanical antiallodynic and thermal antihyperalgesic effects produced by the intraperitoneal administration of 10 mg/kg of CoPP alone or combined with the subplantar administration of a low dose of DPDPE (50 μg) or JWH-015 (30 μg) in the contralateral and ipsilateral paw of CFA-injected animals (n = 6 animals per group).

In another set of experiments, we evaluated the mechanical antiallodynic and thermal antihyperalgesic effects produced by the subplantar administration of 290 μg of SnPP alone or combined with the subplantar administration of a high dose of DPDPE (150 μg) or JWH-015 (300 μg) in the contralateral and ipsilateral paw of CFA-injected animals (n = 6 animals per group).

The doses of CoPP and SnPP combined with DPDPE or JWH-015 were selected in accordance to previous studies (Hervera et al., Anesthesiology, 2013, 118:1180-1197; Hervera et al., Psychopharmacology, 2013, 228:463-477). The doses of all tested opioid and cannabinoid receptor agonists subplantarly administered were chosen from the dose-response curves performed in this study, as the ones that produced a minimal or a maximal antinociceptive effect in CF A-injected mice.

The reversibility of the antinociceptive effects produced by the subplantar administration of DPDPE (150 μg) or JWH-015 (300 μg), as doses that produce the maximal antiallodynic and antihyperalgesic effects after peripheral inflammation, by their subplantar coadministration with specific (naltrindole; 50 μg; AM630; 60 μg) and an unspecific peripheral opioid antagonist (naloxone methiodide, NX-ME; 50 μg) or a cannabinoid 1 receptor (CB1R) antagonist (AM251; 150 μg) (Hervera et al., J Pharmacol Exp Ther, 2010, 334: 887-896; Hervera et al., Mol Pain, 2011, 7:25) was also evaluated (n = 6 animals per group). The doses of all tested opioid and cannabinoid receptor antagonists were selected according to our previous data in animals with chronic pain (Hervera et al., Naunyn Schmiedebergs Arch Pharmacol, 2009, 380:345-352; Hervera et al., Psychopharmacology, 2013, 228:463-477; Negrete et al., PLoS One, 2011, 6:e26688).

Finally, in another set of experiments we evaluated the effects of CoPP on the expression of HO-1, DOR and CB2R in the ipsilateral site of the dorsal root ganglia from CFA-injected mice, by using Western blot assay. In these experiments mice treated with vehicle have been used as controls (n = 5 samples per group). The total number of animals used in this study was 291.

Drugs

CoPP (cobalt (III) protoporphyrin IX chloride) and SnPP (tin (IV) protoporphyrin IX dichloride) were purchased from Frontier scientific (Livchem GmbH & Co, Frankfurt, Germany). DPDPE ([2-D-penicillamine,5-D-penicillamine]-enkephalin), naltrindole and NX-ME (naloxone methiodide) were acquired from Sigma. JWH-015 ((2-Methyl-1-propyl-1H-indol-3-yl)-1-naphthalenylmethanone), AM630 (6-iodo-2-methyl-1-[2-(4-morpholinyl)ethyl]-1H-indol-3-yl](4-methoxyphenyl)methanone) and AM251 (N-(piperidin-1-yl)-5-(4-iodophenyl)-1-(2,4-dichlorophenyl)-4-methyl-1 H-pyrazole-3-carboxamide) were purchased from Tocris (Ellisville, MI).

CoPP and SnPP were dissolved in DMSO (1 % solution in saline). JWH-015, AM630, AM251 were dissolved in DMSO (50 % solution in saline). DPDPE, NX-ME, and naltrindole were dissolved in saline solution (0.9 % NaCl). All drugs were freshly prepared before use. CoPP was intraperitoneally administered 3-4 h before testing in a final volume of 10 ml/kg. SnPP, DPDPE, JWH-015, NX-ME, naltrindole, AM630 and AM251 were administered into the plantar side of the right paw, 30 min before behavioral testing, in a final volume of 30 μl. For each group treated with a drug, the respective control group received the same volume of vehicle.

Statistical analysis

Data are expressed as mean ± SEM. The statistical analysis was performed by using the SPSS (version 17 for Windows, IBM España, Madrid, Spain). All comparisons were run as two-tailed testing.

The mechanical and thermal responses induced by peripheral inflammation in the ipsilateral paw of CFA-injected mice were compared to those effects produced in the contralateral paws of mice by using a paired Student's t test. For each test and drug evaluated, the comparison of the effects produced by the subplantar administration of different doses of DPDPE, JWH-015 or their corresponding vehicle was evaluated by using a one way ANOVA followed by the Student Newman Keuls test.

For each behavioral test, the comparison of the effects produced by the administration of CoPP or SnPP on the local antinociceptive effects produced by DPDPE or JWH-015 was evaluated by using a one way ANOVA followed by the Student Newman Keuls test.

In these experiments, antinociception in von Frey filaments and plantar tests is expressed as the percentage of maximal possible effect, where the test latencies pre (baseline) and postdrug administration are compared and calculated according to the following equation:

$$\text{Maximal possible effect (\%)} = [(\text{drug} - \text{baseline}) / (\text{cut-off} - \text{baseline})] \times 100$$

For each test, the reversal of the local antinociceptive effects produced by DPDPE or JWH-015 with their respective antagonists and the effects produced by these antagonists administered alone were also analyzed by using a one way ANOVA followed by the Student Newman Keuls test.

Changes on the expression of HO-1, DOR and CB2R in the dorsal root ganglia from naive and CFA-injected mice treated with vehicle or CoPP were also analyzed by using a one way ANOVA followed by Student Newman Keuls test. A value of $P < 0.05$ was considered as a significant.

**Example 1. Induction of inflammatory pain.**

The subplantar administration of CFA produced unilateral mechanical allodynia and thermal hyperalgesia (Table 4). Indeed, peripheral inflammation led to a significant decrease of the threshold for evoking paw withdrawal to a mechanical stimulus and a decrease of paw withdrawal latency to thermal stimulus in the ipsilateral paw as compared to the contralateral paw ($P < 0.01$; paired Student's t test).

Table 4. Mechanical response (von Frey filaments strength, g) and thermal response (withdrawal latency, s) in the contralateral and ipsilateral paw of mice after the subplantar administration of CFA. Results are shown as mean values ± SEM; n= 6 animals per experimental group. For each test, * $P < 0.01$ denotes significant differences between ipsilateral and contralateral paw (paired Student's t test).

| Paw | **Mechanical response** von Frey filaments strength (g) | **Thermal response** Withdrawal latency (s) |
|---|---|---|
| Contralateral | 2.4 ± 0.1 | 9.5 ± 0.3 |
| Ipsilateral | 1.4 ± 0.1* | 3.5 ± 0.2* |

**Example 2. Effects of the subplantar administration of DPDPE and JWH-015 on the mechanical allodynia and thermal hyperalgesia induced by peripheral inflammation in mice.**

The subplantar administration of DPDPE (50-150 μg) dose-dependently inhibited the mechanical allodynia (Fig. 1A) and thermal hyperalgesia (Fig. 1B) induced by peripheral inflammation. Indeed, the mechanical antiallodynic and thermal antihyperalgesic effects produced by high doses of DPDPE (75, 100 or 150 μg) in the ipsilateral paw of CFA-injected mice were significantly higher than those produced by a low dose of the same drug or their corresponding vehicle treated animals ($P < 0.001$, one way ANOVA followed by the Student Newman Keuls test).

In a similar way, the subplantar administration of JWH-015 (30-300 μg) also dose-dependently inhibited the mechanical allodynia (Fig. 2A) and thermal hyperalgesia (Fig. 2B) induced by peripheral inflammation. That is, the mechanical antiallodynic and thermal antihyperalgesic effects produced by high doses of JWH-015 (75, 150 or 300 μg) in the ipsilateral paw of CFA-injected mice were significantly higher than those produced by 30 μg of the same drug or their corresponding vehicle treated animals ($P < 0.001$, one way ANOVA followed by the Student Newman Keuls test).

**Example 3. Effects of CoPP on the antiallodynic and antihyperalgesic responses to DPDPE and JWH-015 during peripheral inflammation.**

The effects of the intraperitoneal administration of 10 mg/kg of CoPP on the mechanical antiallodynic and thermal antihyperalgesic effects produced by the subplantar administration of a subanalgesic dose of DPDPE (50 μg), JWH-015 (30 μg) or vehicle in CF A-injected mice were investigated.

For DPDPE, treatment with CoPP also significantly attenuated the mechanical allodynia (Fig. 3A) and thermal hyperalgesia (Fig. 3B) induced by peripheral inflammation in the ipsilateral paw of CFA-injected mice ($P < 0.001$; one way ANOVA versus control vehicle treated mice). Moreover, although treatment with CoPP did not alter the mechanical

antiallodynic (Fig. 3A) effects of DPDPE, it significantly increased the thermal antihyperalgesic (Fig. 3B) effects produced by the subplantar administration of DPDPE in the ipsilateral paw of CFA-injected mice ($P < 0.001$, one way ANOVA versus control group treated with vehicle or DPDPE or CoPP plus vehicle). These results are summarized in Table 5.

Table 5. Mechanical antiallodynic and thermal antihyperalgesic effects produced by the intraperitoneal administration of 10 mg/kg of CoPP alone, the subplantar administration of DPDPE (50 μg) alone, by the combined administration of 10 mg/kg of CoPP and DPDPE (50 μg) and the calculated additive effect of said combination.

| | Experimental results (%) | | | | Calculated result (%) |
|---|---|---|---|---|---|
| | vehicle | DPDPE | CoPP | CoPP + DPDPE | CoPP + DPDPE |
| Mechanical allodynia | 5.8 | 11.8 | 27.1 | 36.7 | 38.9 |
| Thermal hyperalgesia | 4.0 | 16.0 | 26.8 | 61.0 | 42.8 |

Regarding JWH-015, treatment with CoPP significantly enhanced the antiallodynic (Fig. 4A) and antihyperalgesic (Fig. 4B) effects produced by the subplantar administration of JWH-015 in the ipsilateral paw of CFA injected mice ($P < 0.001$, one way ANOVA versus control group treated with vehicle or JWH- 015 or CoPP plus vehicle). These results are summarized in Table 6.

Table 6. Mechanical antiallodynic and thermal antihyperalgesic effects produced by the intraperitoneal administration of 10 mg/kg of CoPP alone, the subplantar administration of JWH-015 (30 μg) alone, by the combined administration of 10 mg/kg of CoPP and JWH-015 (30 μg) and the calculated additive effect of said combination.

| | Experimental results (%) | | | | Calculated result (%) |
|---|---|---|---|---|---|
| | vehicle | JWH-015 | CoPP | CoPP + JWH-015 | CoPP + JWH-015 |
| Mechanical allodynia | 4.1 | 7.5 | 25.3 | 41.9 | 32.8 |
| Thermal | 4.9 | 7.4 | 22.3 | 63.1 | 29.7 |
| hyperalgesia | | | | | |

**Example 4. Effects of the HO-1 inhibitor, tin protoporphyrin IX, on the antinociceptive responses to DPDPE and JWH-015 in CFA-injected mice.**

The effects of the subplantar administration of tin protoporphyrin IX (SnPP) (290 μg) on the mechanical antiallodynic and thermal antihyperalgesic effects produced by the subplantar administration of DPDPE (150 μg) or JWH-015 (300 μg) in CFA-injected mice were assessed.

For DPDPE and JWH-015 and each test evaluated, our results show that while the subplantar administration of SnPP alone did not alter the mechanical allodynia (Fig. 5A) and thermal hyperalgesia (Fig. 5B) induced by peripheral inflammation, its local coadministration with a high dose of DPDPE or JWH-015 significantly decreased the local antiallodynic (Fig. 5A) and antihyperalgesic (Fig. 5B) effects produced by these drugs on the ipsilateral paw of CFA-injected mice ($P < 0.001$, one way ANOVA versus group treated with DPDPE or JWH-015 plus vehicle).

**Example 5. Reversal of the antinociceptive effects of DPDPE and JWH-015 by specific antagonists after peripheral inflammation.**

The antiallodynic and antihyperalgesic effects produced by 150 μg of DPDPE in the ipsilateral paw of CFA-injected mice were completely reversed by its subplantar coadministration with a selective DOR (naltrindole, 50 μg) or a peripheral opioid receptor (NX-ME, 50 μg) antagonist ($P < 0.001$; one way ANOVA, followed by Student Newman Keuls test; Table 7).

The antinociceptive effects produced by 300 μg of JWH-015 in the ipsilateral paw of CFA-injected mice were also completely reversed by its subplantar coadministration with a selective CB2R antagonist (AM630, 60 μg; $P < 0.001$; one way ANOVA, followed by Student Newman Keuls test). The subplantar administration of AM251 (a selective CB1R antagonist; 150 μg) was unable to revert the local antiallodynic and antihyperalgesic effects produced by JWH-015.

The subplantar administration of the different antagonists alone in the ipsilateral paw of CFA-injected mice (Table 7) as well as in the contralateral paw of these animals (data not shown) did not have any significant effect on the different nociceptive responses evaluated in this study.

Table 7. Effects of the subplantar administration of DPDPE (150 μg) or JWH-015 (300 μg) alone or combined with naltrindole (50 μg) or NX-ME (50 μg) and AM630 (60 μg) or AM251 (150 μg) respectively, on the mechanical allodynia and thermal hyperalgesia induced by the subplantar administration of CFA in the ipsilateral paw of mice. For each test and drug tested. * $P < 0.05$ denotes significant differences vs. their respective vehicle plus vehicle treated group (one way ANOVA, followed by the Student Newman Keuls test). Results are shown as mean values ± SEM; n= 6 animals per experimental group.

| | Treatment | | **Mechanical allodynia** von Frey filaments strength (g) | **Thermal hyperalgesia** Withdrawal latency (s) |
|---|---|---|---|---|
| DOR agonist | vehicle | Vehicle | 1.3 ± 0.1 | 3.4 ± 0.5 |
| | DPDPE | Vehicle | 2.4 ± 0.1* | 9.2 ± 0.7* |
| | | Naltrindole | 1.2 ± 0.2 | 3.7 ± 0.4 |
| | | NX-ME | 1.1 ± 0.1 | 3.5 ± 0.2 |
| | vehicle | Naltrindole | 1.3 ± 0.1 | 3.4 ± 0.6 |
| | | NX-ME | 1.2 ± 0.1 | 3.6 ± 0.5 |
| CB2R agonist | vehicle | Vehicle | 1.4 ± 0.1 | 3.8 ± 0.3 |
| | JWH-015 | Vehicle | 2.5 ± 0.2* | 9.2 ± 0.2* |
| | | AM630 | 1.2 ± 0.1 | 4.1 ± 0.4 |
| | | AM251 | 2.4 ± 0.1* | 9.0 ± 0.3* |
| | vehicle | AM630 | 1.3 ± 0.1 | 3.7 ± 0.2 |
| | | AM251 | 1.4 ± 0.1 | 3.6 ± 0.4 |

**Example 6. Effect of CoPP treatment on HO-1, DOR and CB2R protein expression in the dorsal root ganglia from CFA-injected mice.**

The protein levels of HO-1, DOR and CB2R in the dorsal root ganglia from CFA-injected mice treated with vehicle or CoPP as well as from control mice treated with vehicle are shown in Figs. 6-8.

The results show that the dorsal root ganglia expression of HO-1 (Fig. 6) was significantly increased by CoPP treatment ($P < 0.001$; one-way ANOVA versus control vehicle and CFA-injected mice treated with vehicle). Moreover, the decreased protein levels of DOR in the dorsal root ganglia from CFA-injected mice were avoided by CoPP treatment (Fig. 7; $P < 0.001$; one-way ANOVA versus control mice and CFA-injected mice treated with CoPP), while the unchanged peripheral expression of CB2R in the dorsal root ganglia from CFA-injected mice remains unaltered by CoPP treatment (Fig. 8).

In this study, we demonstrated that treatment with an HO-1 inducer compound (CoPP) increased the antinociceptive effects produced by a DOR or CB2R agonist by unaltering CB2R and avoiding the decreased protein levels of DOR induced by peripheral inflammation.

The results further demonstrated that the administration of CoPP (an HO-1 inducer) also inhibited the mechanical and thermal hypersensitivity induced by chronic peripheral inflammation in mice.

Our study also reveals, for first time, that treatment with CoPP significantly enhanced the antinociceptive effects produced by DPDPE and JWH-015 after chronic peripheral inflammation. Moreover, the peripheral antiallodynic and antihyperalgesic effects produced by these drugs were significantly decreased by the subplantar administration of an HO-1 inhibitor, SnPP, indicating that HO-1 participates in the antinociceptive effects produced by a DOR or CB2R agonists during chronic inflammatory pain.

In summary, it has been shown that coadministration of a HO-1 inducer with DOR or CB2R agonists is a strategy to improve their antinociceptive effects during inflammatory pain.

## Claims

**1.** A combination comprising:

(a) at least an heme oxygenase 1 inducer, and
(b) at least a cannabinoid 2 receptor agonist or a selective δ-opioid receptor agonist, for use in the prevention and/or treatment of inflammatory pain.

**2.** Combination for use according to claim 1, wherein the heme oxygenase 1 inducer is selected from the group consisting of cobalt protoporphyrin IX (CoPP), $SnCl_2$, $CoCl_2$, hemin, heme arginate, iron protoporphyrin IX, vitamin B12, 1,2-bis(nicotinamido)propane, 1 -methyl-2-(6-trifluoromethoxy-benzothiazol-2-ylamino)-1H-benzoimidazole-5-carboxylic acid (2-methoxy-ethyl)-amide, 1-methyl-2-(6-trifluoromethoxy-benzo thiazol-2-ylamino)-1H-benzoimidazole-5-carboxylic acid dimethylcarbamoylmethyl-amide, 1-methyl-2-(6-trifluoromethoxy-benzothiazo 1-2-ylamino)-1H-benzoimidazole-5-carboxylic acid [2-(2-hydroxy-ethoxy)-ethyl)]-amide and 2-(benzothiazol-2-ylamino)-1H-benzoimidazole-5-carboxylic acid (1H-indazol-6-yl)-amide or a pharmaceutically acceptable salt thereof

**3.** Combination for use according to claim 2, wherein the heme oxygenase 1 inducer is cobalt protoporphyrin IX (CoPP).

**4.** Combination for use according to any one of the preceding claims, wherein the selective δ-opioid receptor agonist is selected from the group consisting of DPDPE, dermenkephalin, deltorphin I, deltorphin II, SNC-80, (+)-BW373U86, DPI-287, DPI-221, TAN-67, RWJ-394674, 7-spiroindanyloxymorphone, DSLET and SNC-162, or a pharmaceutically acceptable salt thereof.

**5.** Combination for use according to claim 4, wherein the selective δ-opioid receptor agonist is DPDPE.

**6.** Combination for use according to any one of the preceding claims, wherein the cannabinoid 2 receptor agonist is selected from the group consisting of JWH-015, JWH-133, AM-1241, HU-308, GW 405833, L-759,633, L-759,656, CB65, GP1a, GP2a, MDA 19 and SER 601, or a pharmaceutically acceptable salt thereof.

**7.** Combination for use according to claim 6, wherein the cannabinoid 2 receptor agonist is JWH-015 or a pharmaceutically acceptable salt thereof

**8.** Combination for use according to any one of the preceding claims, wherein the ratio by weight of (a) the heme oxygenase 1 inducer and (b) the cannabinoid 2 receptor agonist or selective δ-opioid receptor agonist is from 1:0.01 to 1:2.

**9.** Combination for use according to any one of the preceding claims, wherein the inflammatory pain is chronic inflammatory pain.

**10.** Combination for use according to any one of the preceding claims, wherein the administration of (a) the heme oxygenase 1 inducer and (b) the cannabinoid 2 receptor agonist or selective δ-opioid receptor agonist is simultaneous, separate or sequential.

**11.** Combination for use according to any one of the preceding claims, wherein the administration of the cannabinoid 2 receptor agonist or selective δ-opioid receptor agonist is local.

**12.** Combination for use according to any one of the preceding claims comprising a first composition comprising an heme oxygenase 1 inducer and a second composition comprising a cannabinoid 2 receptor agonist or a selective δ-opioid receptor agonist.

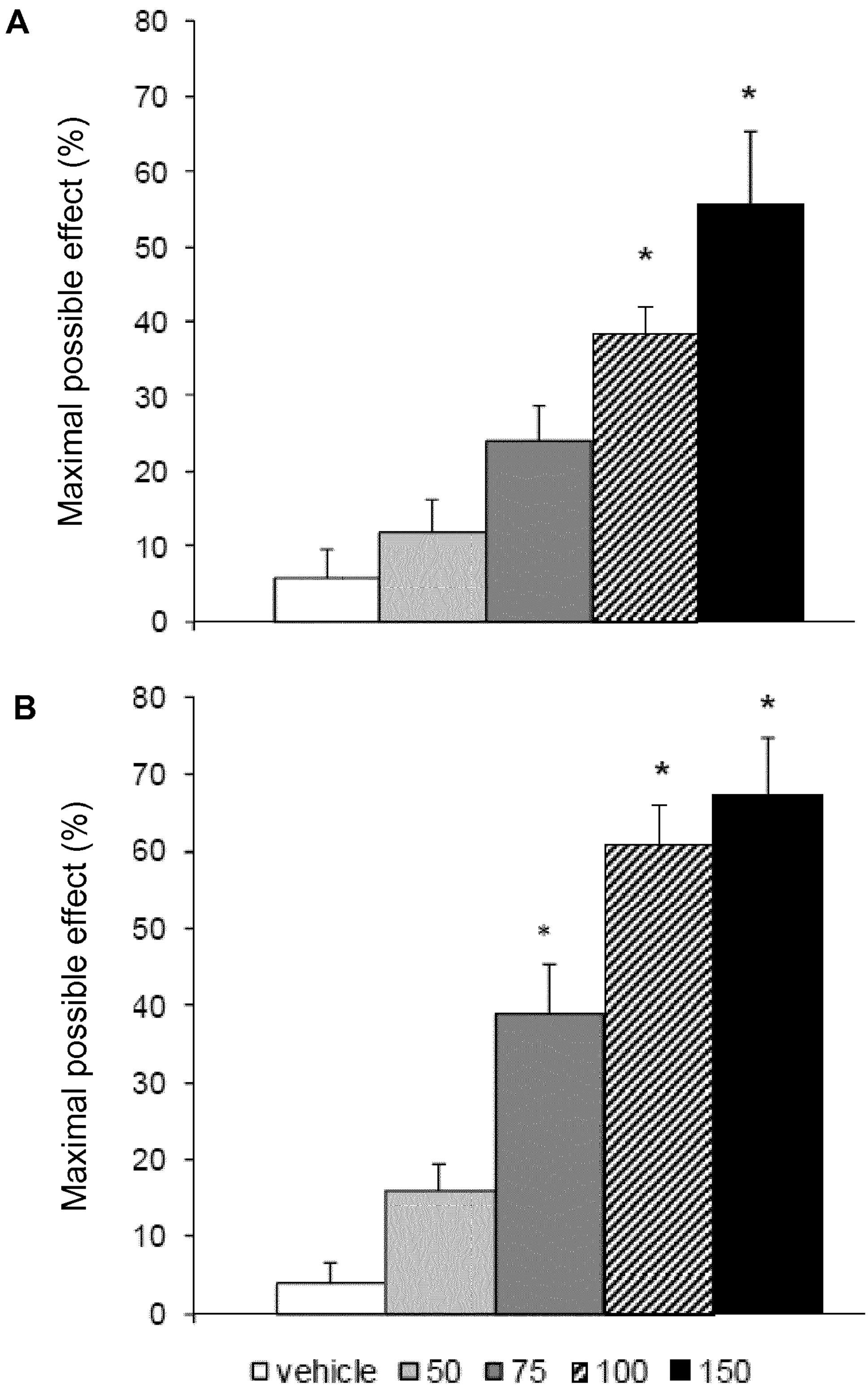
A
B
80
70
60
50
40
30
20
10
0
Maximal possible effect (%)
*
vehicle
50
75
100
150

FIG. 1

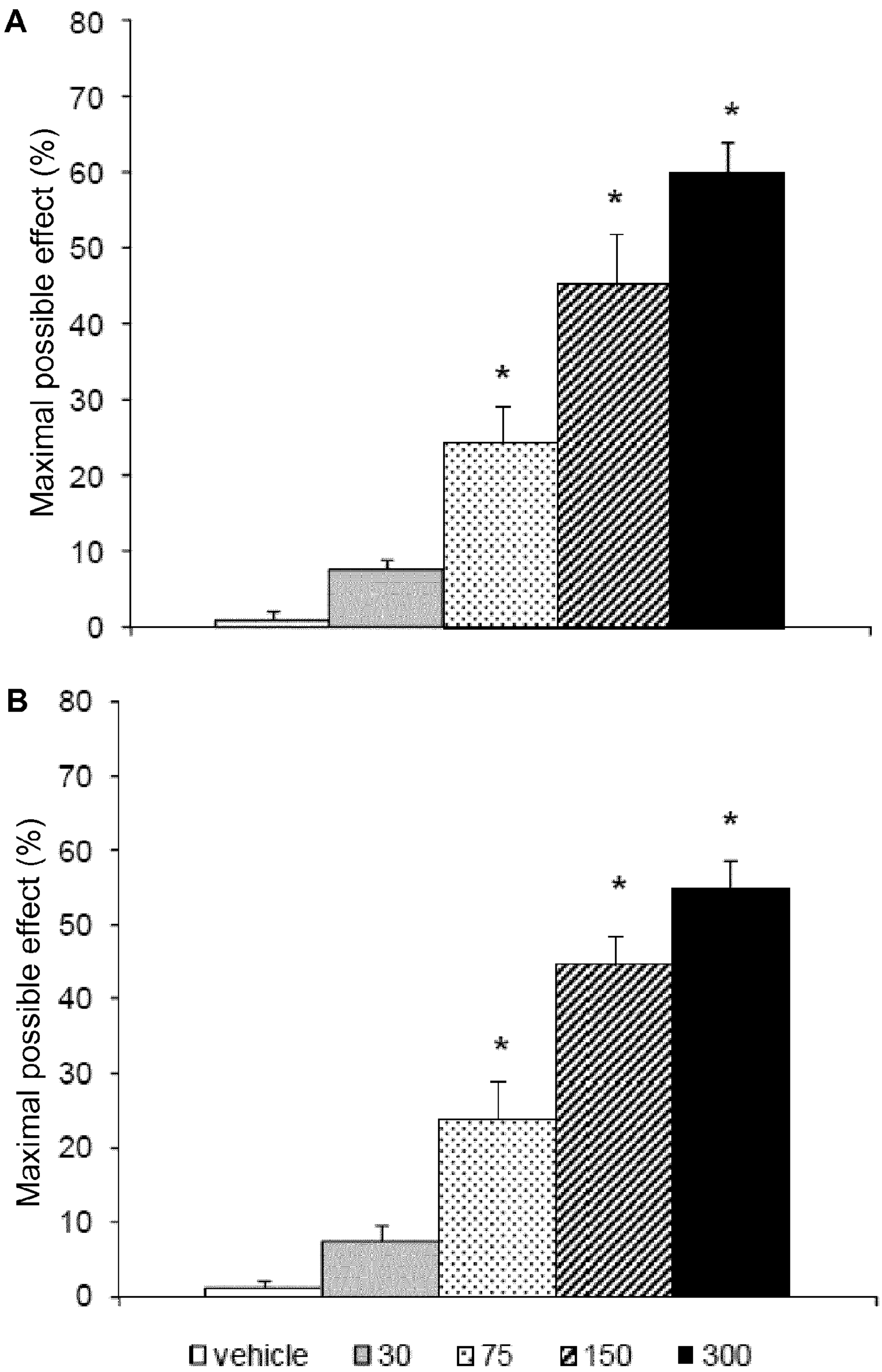
A
B
Maximal possible effect (%)
0
10
20
30
40
50
60
70
80
*
vehicle
30
75
150
300

FIG. 2

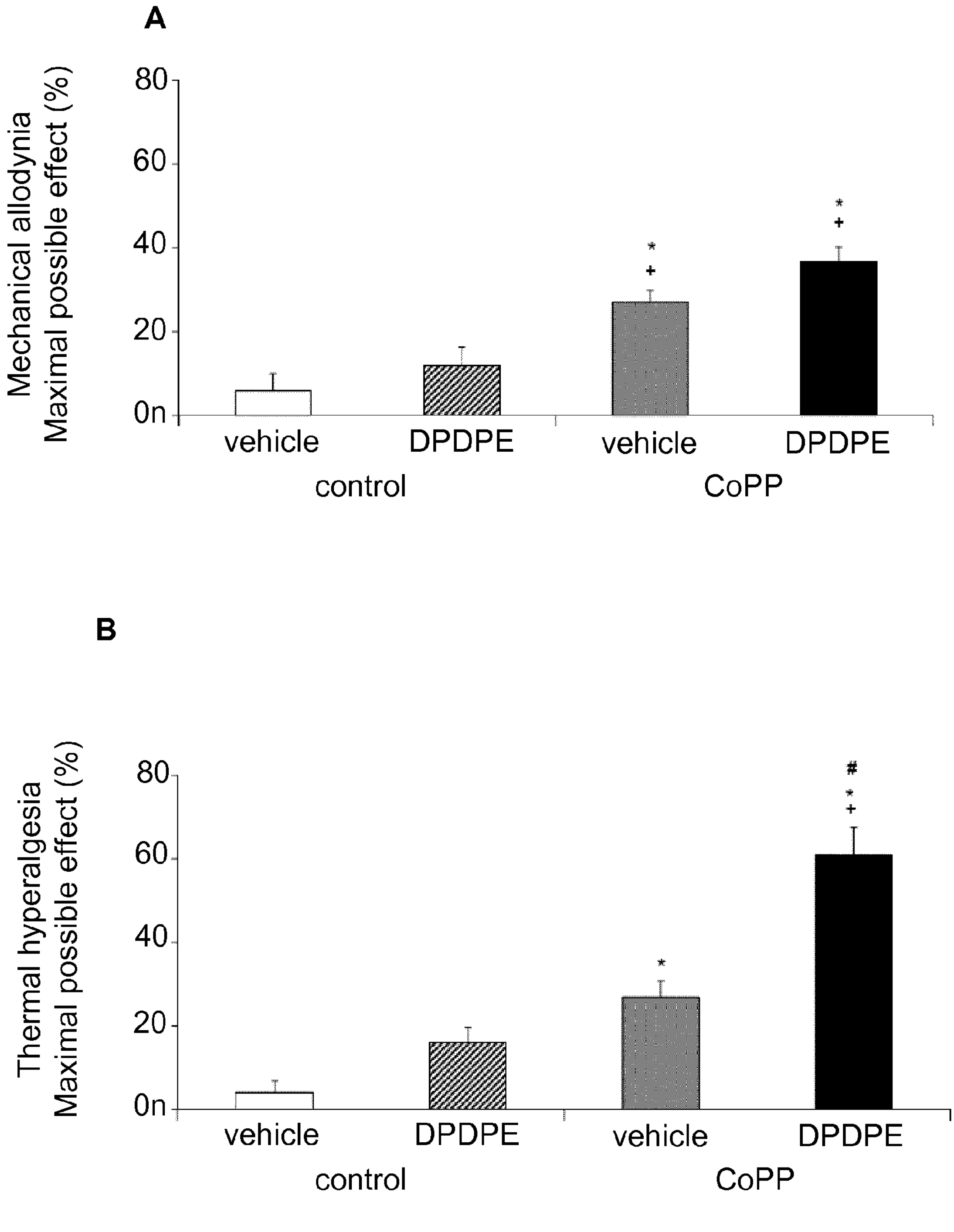
A
Mechanical allodynia
Maximal possible effect (%)
80
60
40
20
0
*
+
*
+
vehicle
DPDPE
vehicle
DPDPE
control
CoPP
B
Thermal hyperalgesia
Maximal possible effect (%)
80
60
40
20
0
*
#
*
+
vehicle
DPDPE
vehicle
DPDPE
control
CoPP

FIG. 3

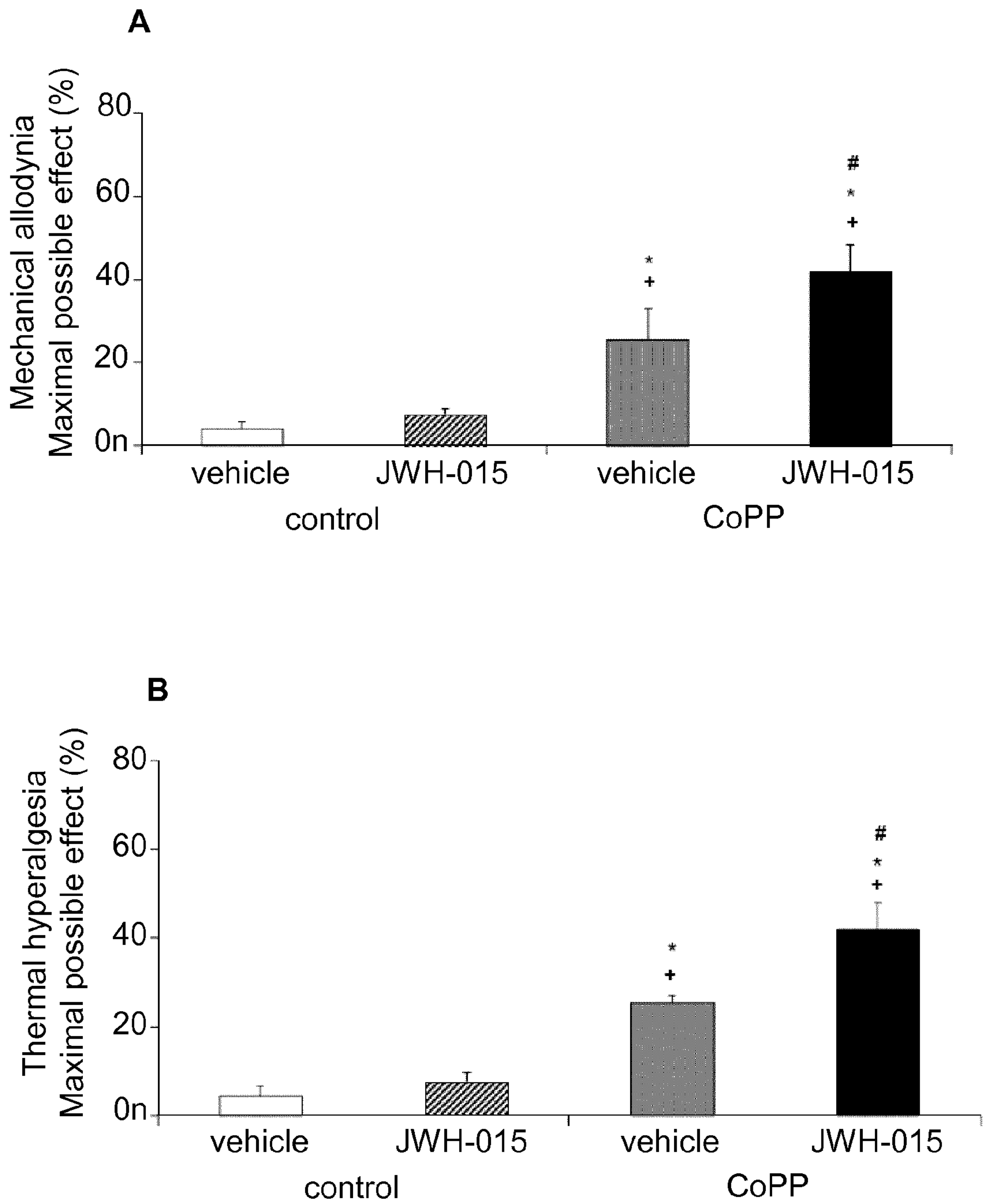
A
Mechanical allodynia
Maximal possible effect (%)
80
60
40
20
0n
#
*
+
vehicle
JWH-015
vehicle
JWH-015
control
CoPP
B
Thermal hyperalgesia
Maximal possible effect (%)
80
60
40
20
0n
vehicle
JWH-015
vehicle
JWH-015
control
CoPP

FIG. 4

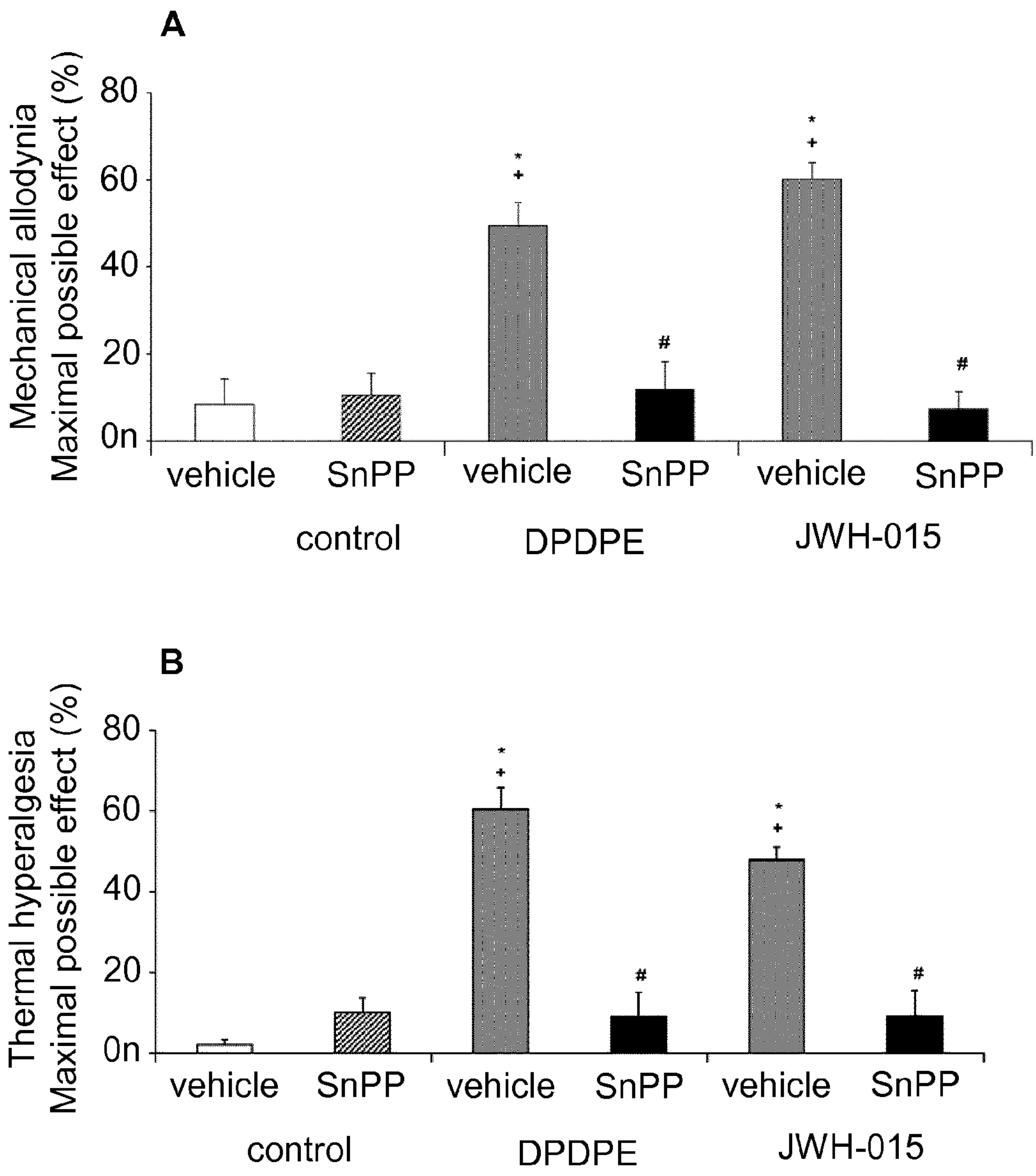
A
Mechanical allodynia
Maximal possible effect (%)
80
60
40
20
0n
*
+
#
vehicle
SnPP
vehicle
SnPP
vehicle
SnPP
control
DPDPE
JWH-015
B
Thermal hyperalgesia
Maximal possible effect (%)
80
60
40
20
0n
vehicle
SnPP
vehicle
SnPP
vehicle
SnPP
control
DPDPE
JWH-015

FIG. 5

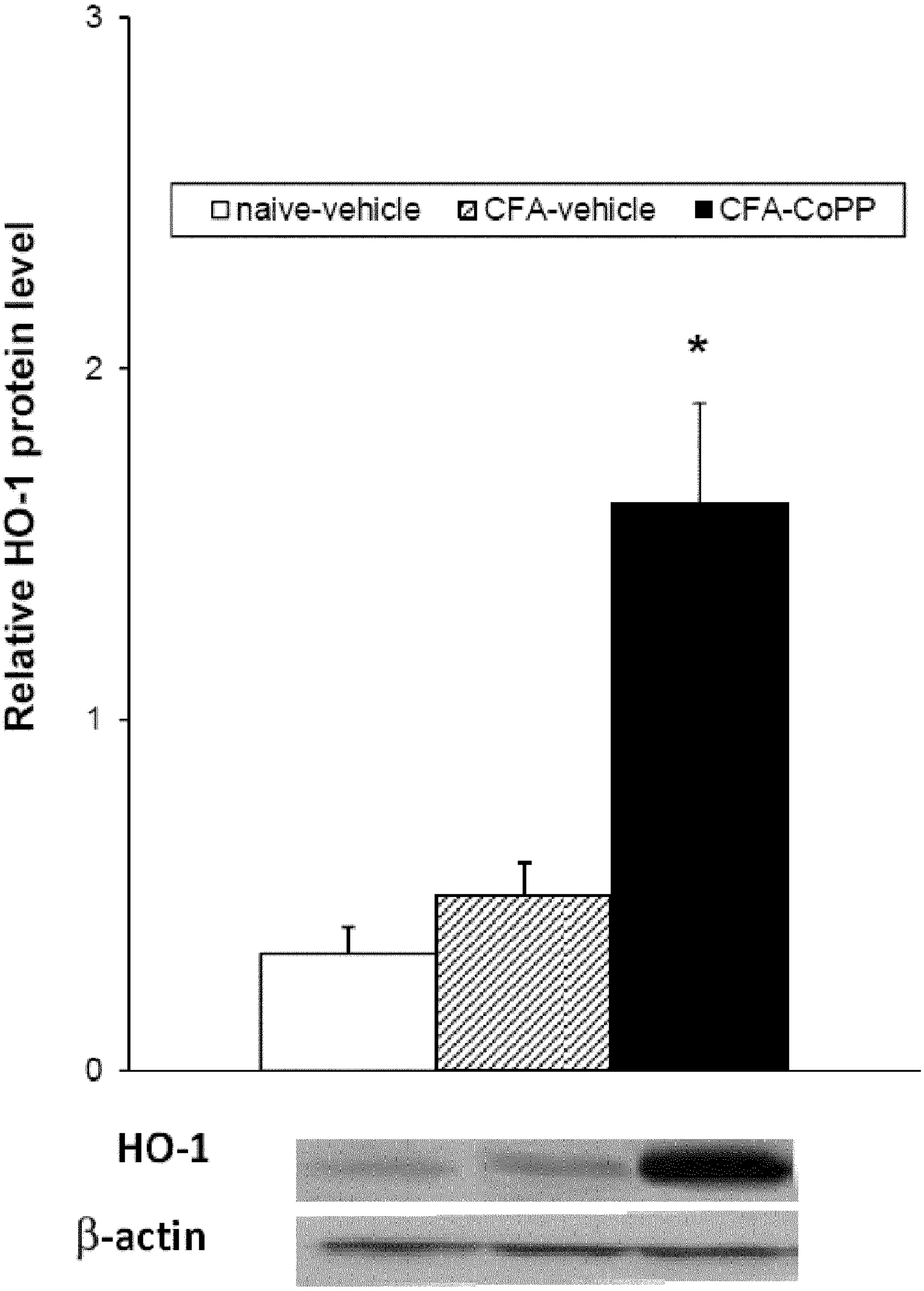
3
2
1
0
Relative HO-1 protein level
naive-vehicle
CFA-vehicle
CFA-CoPP
*
HO-1
β-actin

FIG. 6

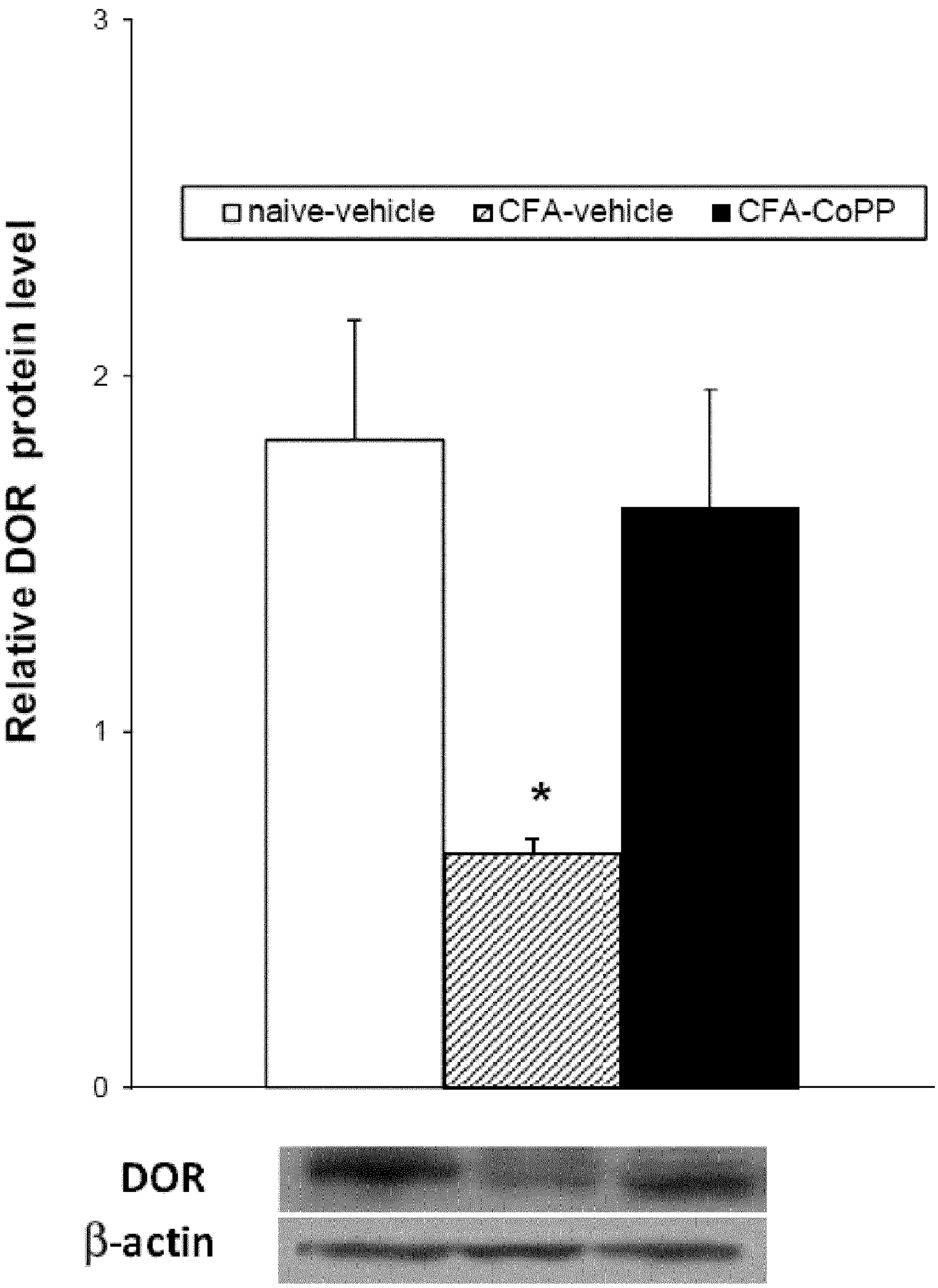
3
2
1
0
Relative DOR protein level
naive-vehicle
CFA-vehicle
CFA-CoPP
*
DOR
β-actin

FIG. 7

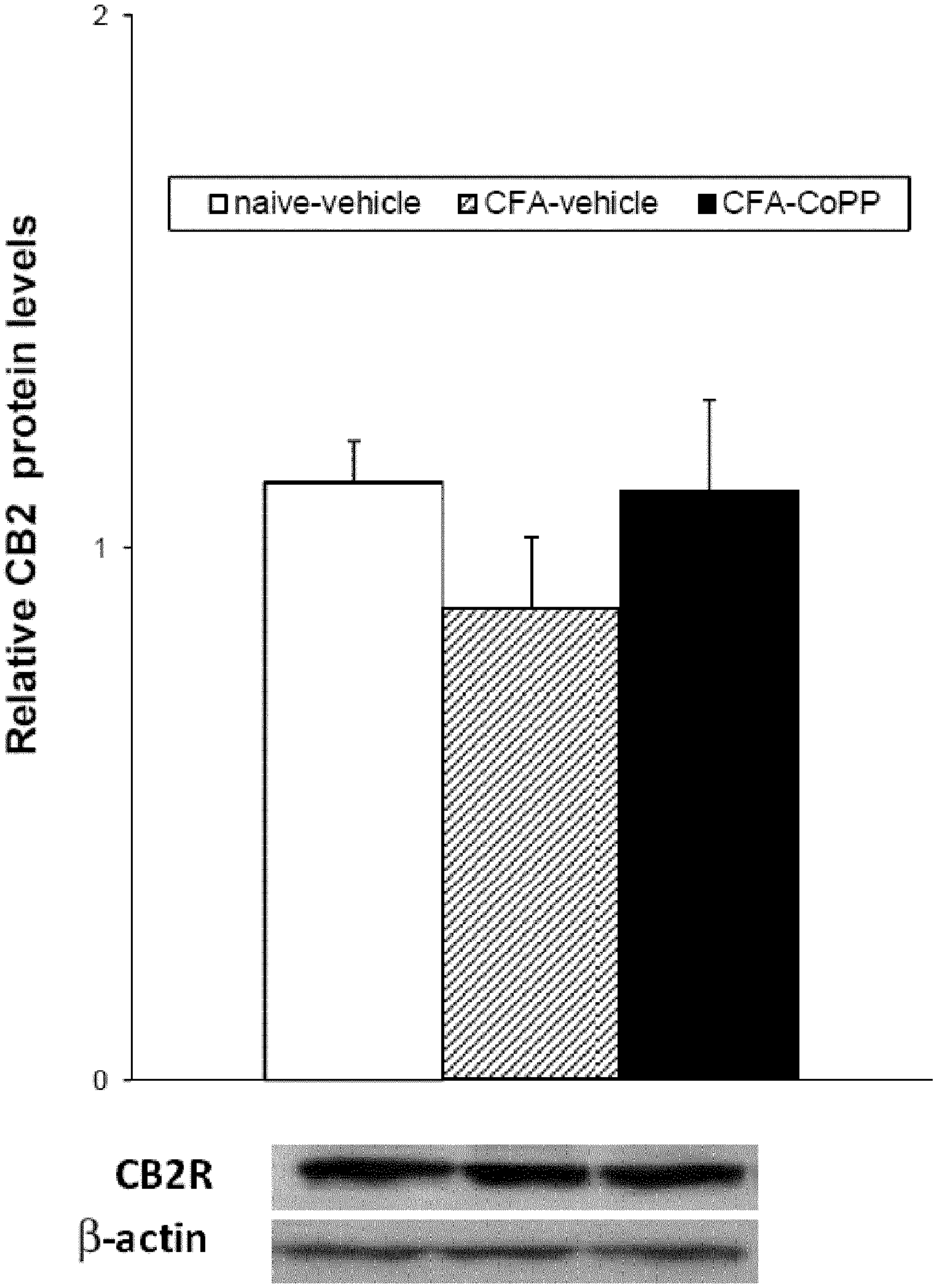
2
1
0
Relative CB2 protein levels
naive-vehicle
CFA-vehicle
CFA-CoPP
CB2R
β-actin

FIG. 8

Europäisches Patentamt
European Patent Office
Office européen des brevets

# EUROPEAN SEARCH REPORT

Application Number
EP 14 18 3542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ROSA A O ET AL: "Nrf2-mediated haeme oxygenase-1 up-regulation induced by cobalt protoporphyrin has antinociceptive effects against inflammatory pain in the formalin test in mice", PAIN, ELSEVIER SCIENCE PUBLISHERS, AMSTERDAM, NL, vol. 137, no. 2, 15 July 2008 (2008-07-15), pages 332-339, XP022765573, ISSN: 0304-3959, DOI: 10.1016/J.PAIN.2007.09.015 [retrieved on 2007-10-26] * the whole document * | 1-3,6-12 | INV. A61K31/404 A61K31/555 A61K33/00 A61K45/06 A61P25/04 A61P29/00 |
| Y | ROGER NEGRETE ET AL: "The Antinociceptive Effects of JWH-015 in Chronic Inflammatory Pain Are Produced by Nitric Oxide-cGMP-PKG-KATP Pathway Activation Mediated by Opioids", PLOS ONE, vol. 6, no. 10, 21 October 2011 (2011-10-21), page e26688, XP055167885, DOI: 10.1371/journal.pone.0026688 * the whole document * | 1-3,6-12 | TECHNICAL FIELDS SEARCHED (IPC) A61K |
| Y | WO 01/32169 A1 (YISSUM RES DEV CO [IL]; FRIDE ESTER [IL]; BREUER AVIVA [IL]; HANUS LUM) 10 May 2001 (2001-05-10) * page 9, line 23 - page 10, line 7 * * figure 7 * | 1-3,6, 8-12 | |

-/--

~~The present search report has been drawn up for all claims~~

2

| Place of search | Date of completion of the search | Examiner |
|---|---|---|
| The Hague | 17 February 2015 | Herdemann, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 3542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BARTOSZYK G D ET AL: "B-vitamins potentiate the antinociceptive effect of diclofenac in carrageenin-induced hyperalgesia in the rat tail pressure test", NEUROSCIENCE LETTERS, LIMERICK, IE, vol. 101, no. 1, 5 June 1989 (1989-06-05), pages 95-100, XP025439458, ISSN: 0304-3940, DOI: 10.1016/0304-3940(89)90447-3 [retrieved on 1989-06-05] * abstract * * page 95, paragraph 1 * ----- | 1,2,6-12 | |
| Y | G. YANG ET AL: "Unique effects of zinc protoporphyrin on HO-1 induction and apoptosis", BLOOD, vol. 97, no. 5, 1 March 2001 (2001-03-01), pages 1306-1313, XP055168211, ISSN: 0006-4971, DOI: 10.1182/blood.V97.5.1306 * abstract * ----- | 1,6-12 | TECHNICAL FIELDS SEARCHED (IPC) |
| Y | ALEXANDRE LOUVET ET AL: "Cannabinoid CB2 receptors protect against alcoholic liver disease by regulating Kupffer cell polarization in mice", HEPATOLOGY, vol. 54, no. 4, 6 September 2011 (2011-09-06), pages 1217-1226, XP055168205, ISSN: 0270-9139, DOI: 10.1002/hep.24524 * abstract * * page 1223, left-hand column, last paragraph * * figure 6 * ----- -/-- | 1,6-12 | |

~~The present search report has been drawn up for all claims~~

2

| Place of search | Date of completion of the search | Examiner |
|---|---|---|
| The Hague | 17 February 2015 | Herdemann, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 3542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HERVERA ARNAU ET AL: "Effects of treatment with a carbon monoxide-releasing molecule and a heme oxygenase 1 inducer in the antinociceptive effects of morphine in different models of acute and chronic pain in mice", PSYCHOPHARMACOLOGY, SPRINGER VERLAG, BERLIN, DE, vol. 228, no. 3, 13 March 2013 (2013-03-13), pages 463-477, XP035370206, ISSN: 0033-3158, DOI: 10.1007/S00213-013-3053-5 [retrieved on 2013-03-13] * abstract * * figure 8 * * page 474, right-hand column, last paragraph - page 476, left-hand column, paragraph 1 * ----- | 1-3,6-12 | |
| A | A HERVERA ET AL: "Treatment with Carbon Monoxide-releasing Molecules and an HO-1 Inducer Enhances the Effects and Expression of [mu]-Opioid Receptors during Neuropathic Pain", ANESTHESIOLOGY, vol. 118, no. 5, 1 May 2013 (2013-05-01), pages 1180-1197, XP055167849, ISSN: 0003-3022, DOI: 10.1097/ALN.0b013e318286d085 * the whole document * ----- | 1-3,6-12 | TECHNICAL FIELDS SEARCHED (IPC) |

2

~~The present search report has been drawn up for all claims~~

| Place of search | Date of completion of the search | Examiner |
|---|---|---|
| The Hague | 17 February 2015 | Herdemann, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 14 18 3542

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

6, 7(completely); 1-3, 8-12(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 14 18 3542

The Search Division considers that the present European patent application does not comply with the requirements of unity of invention and relates to several inventions or groups of inventions, namely:

1. claims: 6, 7(completely); 1-3, 8-12(partially)

   A combination comprising: (a) at least an heme oxygenase 1 inducer, and (b) at least a cannabinoid 2 receptor agonist, for use in the prevention and/or treatment of inflammatory pain.

   ---

2. claims: 4, 5(completely); 1-3, 8-12(partially)

   A combination comprising: (a) at least an heme oxygenase 1 inducer, and (b) at least a selective delta-opioid receptor agonist, for use in the prevention and/or treatment of inflammatory pain.

   ---

# ANNEX TO THE EUROPEAN SEARCH REPORT ON EUROPEAN PATENT APPLICATION NO. EP 14 18 3542

This annex lists the patent family members relating to the patent documents cited in the above-mentioned European search report. The members are as contained in the European Patent Office EDP file on
The European Patent Office is in no way liable for these particulars which are merely given for the purpose of information.

17-02-2015

| Patent document cited in search report | Publication date | Patent family member(s) | Publication date |
|---|---|---|---|
| WO 0132169 A1 | 10-05-2001 | AT 286389 T | 15-01-2005 |
| | | AU 778149 B2 | 18-11-2004 |
| | | AU 1245801 A | 14-05-2001 |
| | | CA 2385928 A1 | 10-05-2001 |
| | | DE 60017287 D1 | 10-02-2005 |
| | | DE 60017287 T2 | 08-12-2005 |
| | | EP 1244440 A1 | 02-10-2002 |
| | | HU 0203681 A2 | 28-04-2003 |
| | | IL 132661 A | 26-11-2008 |
| | | IL 148796 A | 26-11-2008 |
| | | JP 4231906 B2 | 04-03-2009 |
| | | JP 2004505006 A | 19-02-2004 |
| | | KR 20030008208 A | 24-01-2003 |
| | | NZ 518054 A | 31-10-2003 |
| | | US 6864291 B1 | 08-03-2005 |
| | | US 2002173528 A1 | 21-11-2002 |
| | | US 2005165118 A1 | 28-07-2005 |
| | | WO 0132169 A1 | 10-05-2001 |
| | | ZA 200202181 A | 28-05-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1266660 A1
- WO 2012094580 A


### Non-patent literature cited in the description


- **OBARA et al.** *Pain,* 2009, vol. 141, 283-291
- **NEGRETE et al.** *PLoS One,* 2011, vol. 6, e26688
- **HERVERA et al.** *Psychopharmacology,* 2013, vol. 228, 463-477
- **LEÁNEZ et al.** *Eur J Pharmacol,* 2009, vol. 602, 41-49
- **ROSA et al.** *Pain,* 2008, vol. 137, 332-339
- **HERVER et al.** *Anesthesiology,* 2013, vol. 118, 1180-1197
- **HERVERA et al.** *Anesthesiology,* 2013, vol. 118, 1180-1197
- **BENALLAOU et al.** *Arthritis Rheum,* 2007, vol. 56, 2585-2594
- **QUOCK et al.** *European Journal of Pharmacology,* 1997, vol. 326, 101-104
- **CHENG ; PRUSOFF.** *Biochem Pharmacol,* 1973, vol. 22, 3099-3108
- **WANG et al.** *Neuroscience Letters,* 2008, vol. 443, 209-212
- **CHENG ; PRUSOFF.** *Biochem Pharmacol.,* 1973, vol. 22, 3099-3108
- **THOMAS et al.** *Br. J. Pharmacol.,* 2007, vol. 150, 613-623
- **THOMAS et al.** *Br J Pharmacol,* 2005, vol. 146, 917-926
- **CHENG ; PRUSOFF.** *Biochem Pharmacol.,* 1973, vol. 22, 3099-31089
- **LEGGETT et al.** *Br. J. Pharmacol.,* 2004, vol. 141, 253-262
- **ABADJI et al.** *J Neurochem.,* 1999, vol. 72, 2032-2038
- **CHENG-PRUSOFF.** *Biochem Pharmacol,* 1973, vol. 22, 3099-31089
- **E.W. MARTIN.** Remington's Pharmaceutical Sciences. 2005
- **HERVERA et al.** *Naunyn Schmiedebergs Arch Pharmacol,* 2009, vol. 380, 345-352
- **CHAPLAN et al.** *J Neurosci Methods,* 1994, vol. 53, 55-63
- **HARGREAVES et al.** *Pain,* 1988, vol. 32, 77-88
- **HERVERA et al.** *J Pharmacol Exp Ther,* 2010, vol. 334, 887-896
- **HERVERA et al.** *Mol Pain,* 2011, vol. 7, 25